(19) Europäisches Patentamt
European Patent Office
Office européen des brevets

(11) Veröffentlichungsnummer: **0 116 853**
**A1**

(12) # EUROPÄISCHE PATENTANMELDUNG

(21) Anmeldenummer: 84100422.9

(22) Anmeldetag: 17.01.84

(51) Int. Cl.³: C 07 D 237/14
A 61 K 31/50

(30) Priorität: 26.01.83 DE 3302442

(43) Veröffentlichungstag der Anmeldung: 29.08.84 Patentblatt 84/35

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Thyes, Marco, Dr.
Thorwaldsenstrasse 1
D-6700 Ludwigshafen(DE)

(72) Erfinder: Rossy, Phillip A., Dr.
39 Forest Drive
Hillsdale, N.J. 07642(US)

(72) Erfinder: Koenig, Horst, Dr.
Pariser Strasse 4
D-6700 Ludwigshafen(DE)

(72) Erfinder: Lehmann, Hans Dieter, Dr.
Im Hefen 15
D-6945 Hirschberg(DE)

(72) Erfinder: Gries, Josef, Dr.
Roemerweg 43
D-6706 Wachenheim(DE)

(72) Erfinder: Lenke, Dieter, Dr.
Kekuleplatz 1
D-6700 Ludwigshafen(DE)

(54) **Neue Pyridazinone, Verfahren zu ihrer Herstellung, diese Verbindungen enthaltende therapeutische Mittel und deren Verwendung.**

(57) 6-Aryl-3(2H)-pyridazinone der Formel I,

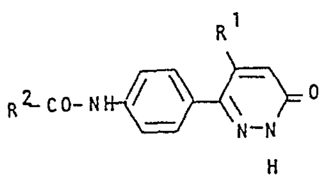

(I)

in der $R^1$ ein Wasserstoffatom oder einen Alkylrest mit 1 bis 3 C-Atomen und $R^2$ ein Wasserstoffatom, einen Alkylrest mit 1 bis 8 C-Atomen, der durch 1 bis 4 Halogenatome, eine Hydroxygruppe, eine Alkyoxygruppe mit 1 bis 4 C-Atomen im Alkyl, eine Alkanoyloxygruppe mit 1 bis 5 C-Atomen im Alkanoylrest oder eine Cyangruppe substituiert ist, einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist, einen Alkenylrest mit 2 bis 8 C-Atomen, eine Gruppe der Formel $-X-R^3$, in der X für ein Sauerstoffatom oder ein Schwefelatom und $R^3$ für einen Alkylrest mit 1 bis 8 C-Atomen, der substituiert sein kann durch 1 bis 4 Halogenatome, eine Alkoxygruppe mit 1 bis 4 C-Atomen im Alkyl, die mindestens 2 C-Atome von X entfernt ist, oder einen Phenylrest, der seinerseits gegebenenfalls 1 bis 3 gleiche oder verschiedene Substituenten aus der Gruppe Alkyl mit 1 bis 3 C-Atomen, Alkoxy mit 1 bis 3 C-Atomen im Alkyl, Halogen, Trifluormethyl oder Nitro aufweist, einen Cycloalkylrest mit 3 bis 8 C-Atomen im Ring, der gegebenenfalls ein- bis vierfach Alkylreste mit 1 bis 4 C-Atomen substituiert ist, einen Alkenylrest mit 3 bis 8 C-Atomen, einen Alkinylrest mit 3 bis 8 C-Atomen oder einen Phenylrest, der gegebenenfalls durch 1 bis 3 gleiche oder verschiedene Reste aus der Gruppe Alkyl mit 1 bis 4 C-Atomen, Alkoxy mit 1 bis 4 C-Atomen im Alkyl, Halogen, Trifluormethyl, Cyan oder Nitro substituiert ist, stehen, oder eine Gruppe der Formel $-NH-R^4$, in der $R^4$ für einen Alkylrest mit 1 bis 8 C-Atomen steht, bedeuten, und $R^2$ zusätzlich, wenn $R^1$ für einen Alkylrest mit 1 bis 3 C-Atomen steht, einen unsubstituierten Alkyrest mit 2 bis 8 C-Atomen bedeutet, Verfahren zu ihrer Herstellung und Arzneimittel daraus.

EP 0 116 853 A1

Neue Pyridazinone, Verfahren zu ihrer Herstellung, diese Verbindungen enthaltende therapeutische Mittel und deren Verwendung

Die Erfindung betrifft neue 6-Aryl-3(2H)-pyridazinone, Verfahren zu ihrer Herstellung, diese Verbindungen enthaltende pharmazeutische Zubereitungen und ihre Verwendung als Arzneimittel bei thrombotischen Erkrankungen und als Antihypertensiva sowie zur Behandlung von Krankheitszuständen, die mit einer pathologisch gesteigerten Magensekretion einhergehen.

Pharmakologisch aktive 6-Aryl-4,5-dihydro-3(2H)-pyridazinone sind bereits mehrfach beschrieben worden.

Beispielsweise werden in der DE-OS 1 670 158 in den Stellungen 4 und 5 unsubstituierte 6-(Acylamino)phenyl-4,5-dihydro-3(2H)-pyridazinone genannt. Als Acylgruppen werden insbesondere Alkanoylreste, Cycloalkylcarbonylreste mit 5 bis 7 C-Atomen im Cycloalkylring, der Acryloylrest und der Methacryloylrest beschrieben. Für diese Verbindungen werden blutdrucksenkende und entzündungshemmende Eigenschaften angegeben.

In der DE-OS 2 150 685 wird das blutdrucksenkend wirkende 6-(p-Formylaminophenyl)-4,5-dihydro-3(2H)-pyridazinon genannt.

Für 6-Phenyl-4,5-dihydro-3(2H)-pyridazinone, die in 4-Stellung eine Alkylgruppe tragen und im Phenylrest in p-Stellung durch eine Gruppe der Formel -NHR', in der R' beispielsweise für einen Acylrest oder einen Ethoxycarbonylrest steht, substituiert sind, werden in der DE-OS 2 304 977 cardiovasculäre und antiphlogistische Eigenschaften angegeben.

In der DE-OS 2 150 436 und den US-PSen 3 824 271 und 3 888 901 werden blutdrucksenkende 5-Alkyl-6-phenyl-4,5-dihydro-3(2H)-pyridazinone, die am Phenylring einen Formylamino- oder einen Alkanoylaminorest tragen, beschrieben.

Aus den DE-OSen 2 727 481 und 2 854 191 sind 6-(p-Alkanoylaminophenyl)-4,5-dihydro-3(2H)-pyridazinone, die in der Alkanoylgruppe durch ein oder mehrere Halogenatome substituiert sind, als Arzneimittel mit thrombozytenaggregationshemmenden und blutdrucksenkenden Eigenschaften bekannt.

In der DE-OS 3 022 176 werden 6-(p-Cycloalkylcarbonylaminophenyl)-4,5-dihydro-3(2H)-pyridazinone beschrieben. Diese können im Cycloalkylrest durch Halogenatome und/oder Alkylreste substituiert sein und besitzen thrombozytenaggregationshemmende und blutdrucksenkende Eigenschaften.
BR/St

In den DE-OSen 3 022 177 und 3 033 702 werden thrombozytenaggregationshemmend und blutdrucksenkend wirkende 6-Phenyl-4,5-dihydro-3(2H)-pyridazinone genannt, deren Phenylrest durch eine Carbamat- oder Thiocarbamat-Gruppe substituiert ist.

Aus der nicht vorveröffentlichten deutschen Patentanmeldung P 3 209 158.3 gehen thrombozytenaggregationshemmend und blutdrucksenkend wirkende 6-(p-Alkenoylaminophenyl)-4,5-dihydro-3(2H)-pyridazinone hervor. Diese können im Alkenoylrest durch ein oder mehrere Halogenatome substituiert sein.

In der DE-OS 2 123 246 werden blutdrucksenkend, coronarerweiternd und antiinflammatorisch wirkende 6-(p-Alkanoylaminophenyl)-4,5-dihydro-3(2H)-pyridazinone, die im Alkanoylrest eine substituierte Aminogruppe tragen, beschrieben.

Für 6-Phenyl-4,5-dihydro-3(2H)-pyridazinone, die im Phenylrest in p-Stellung durch eine Gruppe der Formel -NHCONR"R"', in der die Reste R" und R"' gleich oder verschieden sind und beispielsweise für Wasserstoff, eine Alkylgruppe oder eine Arylgruppe stehen, substituiert sind, werden in der DE-OS 2 157 453 cardiovasculäre und antiinflammatorische Eigenschaften angegeben.

In der DE-OS 2 854 475 werden 2-(Acylamino)phenyl-3,4-diazabicyclo-[4.1.0]hepten-(2)-one-(5) zur Behandlung von thromboembolischen Erkrankungen und zu hohen Blutdrucks vorgeschlagen. Als Acylgruppen werden insbesondere der Formylrest, Alkanoylreste, Halogenalkanoylreste, Cycloalkylcarbonylreste und Alkenoylreste beschrieben. Die Cycloalkylcarbonylreste können im Cycloalkylring durch Halogenatome und/oder Alkylreste substituiert sein.

Schließlich gehen aus der nicht vorveröffentlichten deutschen Patentanmeldung P 3 209 159.1 6-(p-Alkanoylaminophenyl)-4,5-dihydro-3(2H)-pyridazinone und 2-(p-Alkanoylaminophenyl)-3,4-diaza-bicyclo[4.1.0]-hepten-(2)-one-(5) hervor, deren Alkanoylrest in 2-Stellung durch eine Hydroxygruppe, eine Alkoxygruppe, eine Alkanoyloxygruppe oder eine Cyangruppe substituiert ist. Für diese Verbindungen werden thrombozytenaggregationshemmende und blutdrucksenkende Eigenschaften genannt.

Es sind auch bereits 6-(Acylamino)phenyl-3(2H)-pyridazinone bekannt geworden.

Beispielsweise beschreiben W. V. Curran und A. Ross im Journal of Medicinal Chemistry, Band 17 (1974), S. 273-281, das 6-(p-Acetylaminophenyl)-5-methyl-3(2H)-pyridazinon. Herausgestellt wird, daß diese Substanz im Gegensatz zu der entsprechenden 4,5-Dihydro-Verbindung keine blutdrucksenkende Wirkung besitzt.

In der DE-OS 2 810 267 werden 6-(Alkanoylamino)phenyl-3(2H)-pyridazinone genannt. Es soll sich bei diesen Substanzen um Fungizide für die Landwirtschaft sowie um Zwischenprodukte für andere landwirtschaftliche Chemikalien und Arzneimittel handeln.

Aus der DE-OS 2 640 806 gehen 6-(p-Alkanoylaminophenyl)-3(2H)-pyridazinone als Zwischenprodukte für andere Pyridazinone mit fungizider Aktivität hervor.

6-(p-Acetylaminophenyl)-3(2H)-pyridazinon wird von L. Pitarch, R. Coronas und J. Mallol im European Journal of Medicinal Chemistry, Band 9 (1974), S. 644 - 650, sowie von E. A. Steck, R. P. Brundage und L. T. Fletcher im Journal of Heterocyclic Chemistry, Band 11 (1974), S. 755-761, beschrieben. Eine biologische Wirkung wird für diese Verbindung nicht angegeben.

Schließlich werden in der DE-OS 2 647 485 in den Stellungen 4 und 5 unsubstituierte 6-(Acylamino)phenyl-3(2H)-pyridazinone, die im Phenylrest zusätzlich zur Acylaminogruppe ein bis zwei weitere Substituenten anderer Natur tragen können, als Zwischenprodukte für Azopigmente genannt. Als Acylaminogruppe wird insbesondere der 3-Oxobutyrylaminorest beschrieben.

Es wurde nun gefunden, daß 6-Aryl-3(2H)-pyridazinone der allgemeinen Formel I,

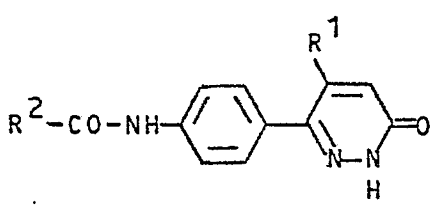

(I)

in der

$R^1$ ein Wasserstoffatom oder einen Alkylrest mit 1 bis 3 C-Atomen und

$R^2$ ein Wasserstoffatom,

einen Alkylrest mit 1 bis 8 C-Atomen, der durch ein bis vier Halogenatome, eine Hydroxygruppe, eine Alkoxygruppe mit 1 bis 4 C-Atomen im Alkyl, eine Alkanoyloxygruppe mit 1 bis 5 C-Atomen im Alkanoylrest oder eine Cyangruppe substituiert ist,

einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist,

einen Alkenylrest mit 2 bis 8 C-Atomen,

eine Gruppe der Formel $-X-R^3$, in der X für ein Sauerstoffatom oder ein Schwefelatom und $R^3$ für einen Alkylrest mit 1 bis 8 C-Atomen, der substituiert sein kann durch ein bis vier Halogenatome, eine Alkoxygruppe mit 1 bis 4 C-Atomen im Alkyl, die mindestens zwei C-Atome von X entfernt ist, oder einen Phenylrest, der seinerseits gegebenenfalls ein bis drei gleiche oder verschiedene Substituenten aus der Gruppe Alkyl mit 1 bis 3 C-Atomen, Alkoxy mit 1 bis 3 C-Atomen im Alkyl, Halogen, Trifluormethyl oder Nitro aufweist, einen Cycloalkylrest mit 3 bis 8 C- Atomen im Ring, der gegebenenfalls ein- bis vierfach durch Alkylreste mit 1 bis 4 C-Atomen substituiert ist, einen Alkenylrest mit 3 bis 8 C-Atomen, einen Alkinylrest mit 3 bis 8 C-Atomen oder einen Phenylrest, der gegebenenfalls durch ein bis drei gleiche oder verschiedene Reste aus der Gruppe Alkyl mit 1 bis 4 C-Atomen, Alkoxy mit 1 bis 4 C-Atomen im Alkyl, Halogen, Trifluormethyl, Cyan oder Nitro substituiert ist, stehen,

oder eine Gruppe der Formel $-NH-R^4$, in der $R^4$ für einen Alkylrest mit 1 bis 8 C-Atomen steht, bedeuten,

und $R^2$ zusätzlich, wenn $R^1$ für einen Alkylrest mit 1 bis 3 C-Atomen steht, einen substituierten Alkylrest mit 2 bis 8 C-Atomen

bedeutet, wertvolle pharmakologische Eigenschaften aufweisen.

Durch ein bis vier Halogenatome, wie Chlor, Brom, Fluor oder Jod, substituierte Alkylreste mit 1 bis 8 C-Atomen für den Rest $R^2$, geradkettig oder verzweigt, sind beispielsweise Chlormethyl, Brommethyl, Fluormethyl, Iodmethyl, 1-Chlorethyl, 1-Bromethyl, 1-Fluorethyl, 1-Iodethyl, 2-Chlorethyl, 2-Bromethyl, 2-Fluorethyl, 2-Iodethyl, 1-Chlorpropyl, 1-Brompropyl, 1-Fluorpropyl, 1-Iodpropyl, 2-Chlorpropyl, 2-Brompropyl, 3-Chlorpropyl, 3-Brompropyl, 3-Fluorpropyl, 1-Chlorisopropyl, 1-Bromisopropyl, 1-Iodisopropyl, 2-Chlorisopropyl, 2-Bromisopropyl, 1-Chlorbutyl, 1-Brombutyl, 1-Fluorbutyl, 4-Chlorbutyl, 4-Brombutyl, 1-Chlorisobutyl, 1-Bromisobutyl, 2-Chlorisobutyl, 1-Chlor-sec-butyl, 1-Brom-sec-butyl, 3-Chlor--sec-butyl, Chlor-tert-butyl, Brom-tert-butyl, 1-Chlorpentyl, 1-Brom-pentyl, 1-Ethyl-1-chlorpropyl, 1-Ethyl-1-brompropyl, Dichlormethyl, Difluormethyl, 1,1-Dichlorethyl, 1,2-Dichlorethyl, 1,2-Dibromethyl, 2,2-Dichlorethyl, 1,1-Dichlorpropyl, 1,2-Dichlorpropyl, 1,2-Dibrompropyl, 1,3-Dichlorpropyl, 2,3-Dibrompropyl, 1,2-Dichlorisopropyl, 1,4-Dichlorbutyl, 1,2-Dibromisobutyl, 1,1-Bis-chlormethyl-ethyl, Trichlormethyl, Trifluormethyl, Chlordifluormethyl und 1,1,2,2-Tetrafluorethyl.

Von den Halogenalkylresten für $R^2$ sind solche bevorzugt, die 1 bis 4 C-Atome und 1 bis 3 Halogenatome, insbesondere Chlor, Brom oder Fluor, enthalten.

Für $R^2$ kommen als geradkettige oder verzweigte Alkylreste mit 1 bis 8 C-Atomen, die durch eine Hydroxygruppe substituiert sind, beispielsweise Hydroxymethyl, 1-Hydroxyethyl, 2-Hydroxyethyl, 1-Hydroxypropyl, 2-Hydroxypropyl, 3-Hydroxypropyl, 1-Hydroxyisopropyl, 2-Hydroxyisopropyl und 1-Hydroxybutyl in Betracht.

Die bevorzugten Hydroxyalkylreste für $R^2$ sind solche, die ein bis vier C-Atome enthalten.

Durch eine Alkoxygruppe mit 1 bis 4 C-Atomen im Alkyl substituierte Alkylreste mit 1 bis 8 C-Atomen für den Rest $R^2$, geradkettig oder verzweigt, sind zum Beispiel Methoxymethyl, Ethoxymethyl, Propoxymethyl, 1-Methoxyethyl, 1-Ethoxyethyl, 1-Propoxyethyl, 2-Methoxyethyl, 2-Ethoxyethyl, 1-Methoxypropyl, 1-Ethoxypropyl, 2-Methoxypropyl, 2-Ethoxypropyl, 3-Methoxypropyl, 3-Ethoxypropyl, 1-Methoxyisopropyl, 1-Ethoxyisopropyl, 2-Methoxyisopropyl, 2-Ethoxyisopropyl, 1-Methoxybutyl und 1-Ethoxybutyl.

Bevorzugte Alkoxyalkylreste für $R^2$ sind solche, die gebildet sind aus der Kombination einer Alkoxygruppe mit 1 bis 3 C- Atomen im Alkyl und einer Alkylengruppe mit 1 bis 4 C-Atomen.

Als geradkettige oder verzweigte Alkylreste mit 1 bis 8 C- Atomen für $R^2$, die durch eine Alkanoyloxygruppe mit 1 bis 5 C-Atomen im Alkanoylrest substituiert sind, seien Acetoxymethyl, Propionyloxymethyl, Butyryloxymethyl, Isobutyryloxymethyl, 1-Acetoxyethyl, 1-Propionyloxyethyl, 1-Butyryloxyethyl, 2-Acetoxyethyl, 2-Propionyloxyethyl, 2-Butyryloxyethyl, 1-Acetoxypropyl, 1-Propionyloxypropyl, 2-Acetoxypropyl, 3-Acetoxypropyl, 1-Acetoxyisopropyl und 1-Acetoxybutyl genannt.

Vorzugsweise sind Alkanoyloxyalkylreste für $R^2$ aus der Kombination einer Alkanoyloxygruppe mit 1 bis 4 C-Atomen im Alkanoylrest und einer Alkylengruppe mit 1 bis 4 C-Atomen gebildet.

Eine Cyangruppe tragende Alkylreste mit 1 bis 8 C-Atomen für den Rest $R^2$, geradkettig oder verzweigt, sind beispielsweise Cyanmethyl, 1-Cyanethyl, 2-Cyanethyl, 1-Cyanpropyl, 2-Cyanpropyl, 3-Cyanpropyl, 1-Cyanisopropyl, 2-Cyanisopropyl, 1-Cyanbutyl, 4- Cyanbutyl, 1-Cyanisobutyl und 5-Cyanpentyl.

Die bevorzugten Cyanalkylreste für $R^2$ sind solche, die gebildet sind aus der Kombination einer Cyangruppe und einer Alkylengruppe mit 1 bis 4 C-Atomen.

Für den Rest $R^2$ kommen als gegebenenfalls ein- bis vierfach durch Halogenatome, wie Chlor, Brom oder Fluor, und/oder Alkylreste mit 1 bis 4 C-Atomen substituierte Cycloalkylgruppen mit 3 bis 8 C-Atomen im Ring zum Beispiel Cyclopropyl, 1-Methylcyclopropyl, 2-Methylcyclopropyl, 2,2-Dimethylcyclopropyl, 2,2,3,3-Tetramethylcyclopropyl, 1-Chlorcyclopropyl, 2-Bromcyclopropyl, 2,2-Dichlorcyclopropyl, 2,2-Dibromcyclopropyl, 2,2-Dichlor-1-methylcyclopropyl, Cyclobutyl, 1-Methylcyclobutyl, 2-Methylcyclobutyl, 3-Methylcyclobutyl, 3,3-Dimethylcyclobutyl, 1-Propylcyclobutyl, 3-Tertiärbutylcyclobutyl, 1-Chlorcyclobutyl, 2-Chlorcyclobutyl, 3-Chlorcyclobutyl, 1-Bromcyclobutyl, 2,2,3,3-Tetrafluorcyclobutyl, 1-Brom-3,3-dimethylcyclobutyl, Cyclopentyl, 1-Methylcyclopentyl, 2,5-Dimethylcyclopentyl, 1-Chlorcyclopentyl, 3,4-Dichlorcyclopentyl, Cyclohexyl und 1-Methylcyclohexyl in Betracht.

Bevorzugte Cycloalkylreste für $R^2$ sind solche, die 3 bis 5 C- Atome im Ring enthalten und gegebenenfalls ein- bis dreifach durch Halogenatome und/oder Methylreste substituiert sind.

Alkenylreste mit 2 bis 8 C-Atomen für den Rest $R^2$ sind beispielsweise Vinyl, Propenyl, Isopropenyl, Allyl, But-1-enyl, But-2-enyl, But-3-enyl, 2-Methyl-prop-1-enyl und Pent-1-enyl.

Von den Alkenylresten für $R^2$ sind solche bevorzugt, die 2 bis 4 C-Atome enthalten.

Als unsubstituierte Alkylreste mit 1 bis 8 C-Atomen für den Rest $R^3$, geradkettig oder verzweigt, seien Methyl, Ethyl, Propyl, Isopropyl, Butyl, Isobutyl, sec-Butyl, tert-Butyl, Pentyl, 2- Methylbutyl und Isopentyl genannt.

Die bevorzugten unsubstituierten Alkylreste für $R^3$ sind solche, die ein bis vier C-Atome enthalten.

Durch ein bis vier Halogenatome, wie Chlor, Brom oder Fluor, substituierte Alkylreste mit 1 bis 8 C-Atomen für den Rest $R^3$, geradkettig oder verzweigt, sind beispielsweise Chlormethyl, Brommethyl, Dichlormethyl, Trifluormethyl, 1-Chlorethyl, 2-Chlorethyl, 2-Bromethyl, 2-Fluorethyl, 1,2-Dichlorethyl, 1,1,2-Trichlorethyl, 2,2,2-Trichlorethyl, 2,2,2-Tribromethyl, 2,2,2-Trifluorethyl, 2-Brompropyl, 3-Chlorpropyl, 2,3-Dichlorpropyl, 2,3-Dibrompropyl, 3-Brom-2-chlorpropyl, 2-Chlorisopropyl, 2,2'-Dichlorisopropyl, 2-Chlorbutyl, 4-Chlorbutyl, 1-Chlormethylpropyl, 3,4-Dibrombutyl, 2-Chlorisobutyl und 3-Chlorisobutyl.

Von den Halogenalkylresten für $R^3$ sind solche bevorzugt, die 1 bis 4 C-Atome und ein bis drei Halogenatome, insbesondere Chlor, Brom oder Fluor, enthalten.

Alkoxyalkylreste für $R^3$, die gebildet sind aus der Kombination einer Alkoxygruppe mit 1 bis 4 C-Atomen im Alkyl und einer geradkettigen oder verzweigten Alkylengruppe mit 2 bis 8 C-Atomen und in denen die Alkoxygruppe so angeordnet ist, daß sie mindestens durch zwei Kohlenstoffatom von X getrennt ist, sind zum Beispiel 2-Methoxyethyl, 2-Ethhoxyethyl, 2-Propoxyethyl, 2-Isopropoxyethyl, 2-Butoxyethyl, 2-Methoxypropyl, 2-Ethoxypropyl, 3-Methoxypropyl, 3-Ethoxypropyl, 2-Methoxyisopropyl, 2-Ethoxyisopropyl, 2-Methoxybutyl, 3-Methoxybutyl, 4-Methoxybutyl und 1,1-Dimethyl2-methoxyethyl.

Vorzugsweise sind die Alkoxyalkylreste für $R^3$ aus der Kombination einer Alkoxygruppe mit 1 bis 3 C-Atomen im Alkyl und einer Alkylengruppe mit 2 bis 4 C-Atomen gebildet.

Durch eine Phenylgruppe substituierte Alkylreste mit 1 bis 8 C-Atomen für den Rest $R^3$, geradkettig oder verzweigt, sind zum Beispiel Benzyl, 1-Phenylethyl, 2-Phenylethyl, 1-Phenylpropyl, 2-Phenylpropyl, 3-Phenylpropyl, 1-Methyl-2-phenylethyl, 1-Methyl-1-phenylethyl und 4-Phenylbutyl.

Die bevorzugten Phenylalkylreste für $R^3$ sind solche, die gebildet sind aus der Kombination einer Phenylgruppe und einer Alkylengruppe mit 1 bis 4 C-Atomen.

Arylalkylreste für $R^3$, die gebildet sind aus der Kombination einer ein bis drei gleiche oder verschiedene Substituenten aus der Gruppe Alkyl mit

1 bis 3 C-Atomen, wie Methyl, Ethyl oder Propyl, Alkoxy mit 1 bis 3 C-Atomen im Alkyl, wie Methoxy oder Ethoxy, Halogen, wie Chlor, Brom oder Fluor, Trifluormethyl oder Nitro tragenden Phenylgruppe und einer geradkettigen oder verzweigten Alkylengruppe mit 1 bis 8 C-Atomen sind beispielsweise o-Methylbenzyl, m-Methylbenzyl, p-Methylbenzyl, p-Ethylbenzyl, o-Methoxybenzyl, m-Methoxybenzyl, p-Methoxybenzyl, o-Ethoxybenzyl, m,p-Dimethoxybenzyl, m,m',p-Trimethoxybenzyl, o-Chlorbenzyl, m-Chlorbenzyl, p-Chlorbenzyl, o-Fluorbenzyl, m-Fluorbenzyl, p-Fluorbenzyl, m-Trifluormethylbenzyl, o-Nitrobenzyl, p-Nitrobenzyl, 1-(m-Methoxyphenyl)-ethyl, 2-(m,p-Dimethoxyphenyl)ethyl, 2-(p-Fluorphenyl)-ethyl und 2-(o-Nitrophenyl)-ethyl.

Vorzugsweise sind die Arylalkylreste für $R^3$ aus der Kombination einer substituierten Phenylgruppe und einer Alkylengruppe mit 1 bis 4 C-Atomen gebildet.

Für den Rest $R^3$ kommen als gegebenenfalls ein- bis vierfach durch Alkylreste mit 1 bis 4 C-Atomen, wie Methyl, Ethyl oder Propyl, substituierte Cycloalkylgruppen mit 3 bis 8 C-Atomen im Ring zum Beispiel Cyclopropyl, 1-Methylcyclopropyl, 2-Methylcyclopropyl, 1-Ethylcyclopropyl, 2,2-Dimethylcyclopropyl, 1,2,2-Trimethylcyclopropyl, Cyclobutyl, 1-Methylcyclobutyl, 2-Methylcyclobutyl, 3-Methylcyclobutyl, 1-Ethylcyclobutyl, 2-Ethylcyclobutyl, 1,2-Dimethylcyclobutyl, 2,2-Dimethylcyclobutyl, 3,3-Dimethylcyclobutyl, Cyclopentyl, 1-Methylcyclopentyl, 2-Methylcyclopentyl, Cyclohexyl und Cycloheptyl in Betracht.

Die unsubstituierten und auch die substituierten Cycloalkylreste für $R^3$ enthalten vorzugsweise 3 bis 6 C-Atome im Ring.

Von den Alkenylresten mit 3 bis 8 C-Atomen, die für $R^3$ stehen können, seien Allyl, But-2-enyl, But-3-enyl, 1-Methylallyl, 2-Methylallyl und Pent-4-enyl genannt.

Bevorzugte Alkenylreste für $R^3$ sind solche mit 3 bis 5 C-Atomen.

Alkinylreste mit 3 bis 8 C-Atomen für $R^3$ sind beispielsweise Prop-2-inyl, But-2-inyl, But-3-inyl, 1-Methylprop-2-inyl, Pent-2-inyl, Pent-4-inyl, 1-Methylbut-2-inyl, 1-Methylbut-3-inyl und 1,1-Dimethylprop-2-inyl.

Die bevorzugten Alkinylreste für $R^3$ sind solche mit 3 bis 5 C-Atomen.

Beispiele für die ein bis drei gleiche oder verschiedene Substituenten aus der Gruppe Alkyl mit 1 bis 4 C-Atomen, wie Methyl, Ethyl oder Propyl, Alkoxy mit 1 bis 4 C-Atomen im Alkyl, wie Methoxy oder Ethoxy, Halogen, wie Chlor, Brom oder Fluor, Trifluormethyl, Cyan oder Nitro tragende Phenylgruppe, die für $R^3$ stehen kann, sind o-Tolyl, m-Tolyl, p-Tolyl, p-Ethylphenyl, o,p-Dimethylphenyl, o,m,p-Trimethylphenyl, o-Methoxyphenyl, m-Methoxyphenyl, p-Methoxyphenyl, o-Ethoxyphenyl, o-Ethoxy-p--ethylphenyl, o-Chlorphenyl, m-Chlorphenyl, p-Chlorphenyl, p-Bromphenyl, p-Fluorphenyl, m,p-Dichlorphenyl, p-Chlor-o-methylphenyl, m-Trifluormethylphenyl, p-Cyanphenyl, o-Nitrophenyl, m-Nitrophenyl und p-Nitrophenyl.

Als Alkylreste mit 1 bis 8 C-Atomen für den Rest $R^4$ in der Gruppe der Formel $-NH-R^4$, geradkettig oder verzweigt, kommen die gleichen Reste in Betracht wie beim Rest $R^3$ in der Gruppe der Formel $-X-R^3$.

Vorzugsweise enthalten die Alkylreste für den Rest $R^4$ in der Gruppe der Formel $-NH-R^4$ 1 bis 4 C-Atome.

Die unsubstituierten Alkylreste mit 2 bis 8 C-Atomen für den Rest $R^2$ können geradkettig oder verzweigt sein. Beispiele hierfür sind Ethyl, Propyl, Isopropyl, Butyl, Isobutyl, sec-Butyl, tert-Butyl, Pentyl, 2-Methylbutyl und Isopentyl.

Die bevorzugten unsubstituierten Alkylreste für den Rest $R^2$ sind solche mit 2 bis 4 C-Atomen.

Im Hinblick auf $R^1$ sind bevorzugte Verbindungen die, in denen $R^1$ für ein Wasserstoffatom oder eine Methylgruppe steht.

Zu den neuen Verbindungen der Formel I, in denen $R^2$ für ein Wasserstoffatom, einen Alkylrest mit 1 bis 8 C-Atomen, der durch ein bis vier Halogenatome, eine Alkoxygruppe mit 1 bis 4 C-Atomen im Alkyl, eine Alkanoyloxygruppe mit 1 bis 5 C-Atomen im Alkanoylrest oder eine Cyangruppe substituiert ist, einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist, einen Alkenylrest mit 2 bis 8 C-Atomen oder (wenn $R^1$ $C_{1-3}$-Alkyl bedeutet) einen Alkylrest mit 2 bis 8 C-Atomen steht, kann man gelangen, indem man eine Verbindung der Formel II,

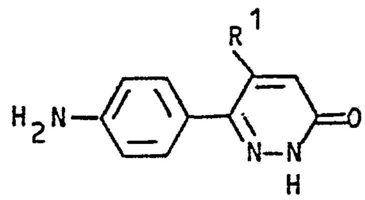

(II)

in der $R^1$ die für Formel I angegebenen Bedeutungen hat, mit einem Acylierungsmittel der Formel III,

$R^5$-CO-Y (III)

in der $R^5$ ein Wasserstoffatom, einen Alkylrest mit 1 bis 8 C-Atomen, der durch ein bis vier Halogenatome, eine Alkoxygruppe mit 1 bis 4 C-Atomen im Alkyl, eine Alkanoyloxygruppe mit 1 bis 5 C-Atomen im Alkanoylrest oder eine Cyangruppe substituiert ist, einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist, einen Alkenylrest mit 2 bis 8 C-Atomen oder (wenn $R^1 = C_{1-3}$-Alkyl) einen Alkylrest mit 2 bis 8 C-Atomen bedeutet, und Y für OH, ein Halogenatom, insbesondere ein Chloratom, einen niederen Alkoxyrest oder $-O-CO-R^5$, wobei $R^5$ die für Formel III angegebenen Bedeutungen hat, steht, in an sich bekannter Weise umsetzt.

Gemäß den für Y angegebenen Bedeutungen sind zweckmäßige Acylierungsmittel die entsprechenden Carbonsäuren, Carbonsäurehalogenide, insbesondere Chloride, Carbonsäureester, insbesondere Methyl- und Ethylester, und die entsprechenden Carbonsäureanhydride.

Die Acylierung wird unter an sich üblichen Bedingungen durchgeführt, in der Regel unter Verwendung von wenigstens einer äquimolaren Menge des Acylierungsmittels, zweckmäßig in Gegenwart eines Lösungsmittels, gegebenenfalls in Gegenwart eines säurebindenden Mittels, bei einer ausreichenden Temperatur von im allgemeinen bis zu 100 °C, bevorzugt bis zu 80 °C, gegebenenfalls bei der Siedetemperatur des Reaktionsgemisches, und gegebenenfalls unter Anwendung von Druck.

Als Lösungsmittel kommen unter den Reaktionsbedingungen inerte Lösungsmittel, wie aromatische Kohlenwasserstoffe, beispielsweise Toluol oder Xylol, cyclische aliphatische Ether, zum Beispiel Tetrahydrofuran oder Dioxan, aliphatische oder aromatische Chlorkohlenwasserstoffe, beispielsweise Methylenchlorid, Ethylenchlorid oder Chlorbenzol, aliphatische Ketone, zum Beispiel Aceton, Diethylketon oder Methylethylketon, oder

Dialkylformamide, insbesondere Dimethylformamid, in Betracht. Säurebindende Mittel sind zweckmäßigerweise schwache anorganische Basen, wie Natrium- oder Kaliumcarbonat, Natrium- oder Kaliumhydrogencarbonat, oder organische Basen, z. B. tertiäre Amine wie Triethylamin.

Die neuen Verbindungen der Formel I, die Alkohole darstellen, d. h. in denen $R^2$ für einen Hydroxyalkylrest steht, lassen sich herstellen, indem man eine Verbindung der Formel I, in der $R^2$ einen Alkanoyloxyalkylrest bedeutet, nach an sich bekannten Methoden, z. B. mit einer Base wie Natriumhydroxid, in einem Gemisch aus Wasser und einem niederen Alkohol, wie Methanol, Ethanol oder Propanol, selektiv hydrolysiert. Gearbeitet wird hierbei bei Temperaturen von 0 bis 50 °C, bevorzugt bei Raumtemperatur. Die hier verwendeten Ausgangsverbindungen werden - wie oben angegeben - durch Behandlung einer Verbindung der Formel II mit einem Acylierungsmittel der Formel III, in dem $R^5$ für eine Alkanoyloxygruppe steht, erhalten.

Die neuen Verbindungen der Formel I, in denen $R^2$ für eine Gruppe der Formel $-X-R^3$ steht, können erhalten werden, indem man ein Aminophenyl-pyridazinon der Formel II,

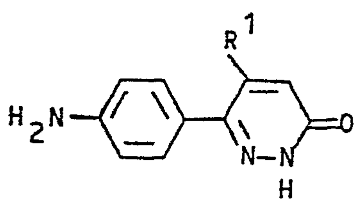

(II)

in der $R^1$ die für Formel I angegebenen Bedeutungen hat, mit einer Verbindung der Formel IV,

$$R^3-X-CO-Z \qquad (IV)$$

in der X und $R^3$ die gleichen Bedeutungen haben wie in der Gruppe der Formel $-X-R^3$, die für den Rest $R^2$ der Verbindungen der Formel I stehen kann, und Z für ein Halogenatom, insbesondere Chlor, steht, in an sich bekannter Weise umsetzt.

Zur Umsetzung einer Verbindung der Formel II mit den Halogenameisensäureestern bzw. Halogenthioameisensäure-S-estern der Formel IV wird unter den oben für die Acylierung einer Verbindung der Formel II mit einem Carbonsäurehalogenid der Formel III (Y = Halogen) angegebenen Bedingungen gearbeitet.

0116853

Die neuen Pyridazinone der Formel I, in denen $R^2$ für eine Gruppe der Formel $-NH-R^4$ steht, lassen sich herstellen, indem man eine Verbindung der Formel II,

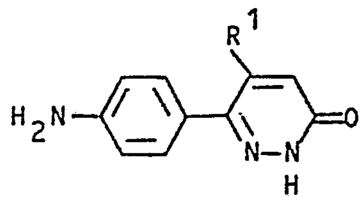

(II)

in der $R^1$ die für Formel I angegebenen Bedeutungen hat, mit einem Isocyanat der Formel V,

$$R^4-N=C=O \qquad (V)$$

in der $R^4$ die gleiche Bedeutung hat wie in der Gruppe der Formel $-NH-R^4$, die für den Rest $R^2$ der Verbindungen der Formel I stehen kann, in an sich bekannter Weise umsetzt.

Die Umsetzung eines Aminophenyl-pyridazinons der Formel II mit einem Isocyanat der Formel V wird unter an sich üblichen Bedingungen durchgeführt. In der Regel unter Verwendung von wenigstens einer äquimolaren Menge einer Verbindung der Formel V, zweckmäßig in Gegenwart eines Lösungsmittels, gegebenenfalls unter Zusatz eines der zur Beschleunigung von Isocyanatreaktionen üblicherweise eingesetzten Katalysatoren, wie Triethylamin, bei einer ausreichenden Temperatur von im allgemeinen bis zu 100 °C, bevorzugt bis zu 80 °C, gegebenenfalls bei der Siedetemperatur des Reaktionsgemisches, und gegebenenfalls unter Anwendung von Druck.

Als Lösungsmittel können die gleichen Lösungsmittel verwendet werden wie bei der oben beschriebenen Umsetzung eines Aminophenylpyridazinons der Formel II mit einem Acylierungsmittel der Formel III. Ist das Isocyanat der Formel V flüssig, so kann die Reaktion auch in überschüssigem V als Lösungsmittel durchgeführt werden.

Eine weitere Herstellungsmöglichkeit für diejenigen neuen Verbindungen der Formel I, in denen $R^2$ für einen Alkylrest mit 1 bis 8 C-Atomen steht, der durch eine Alkoxygruppe mit 1 bis 4 C-Atomen im Alkyl oder eine Cyangruppe substituiert ist, besteht darin, daß man in einer Verbindung der Formel I, in der $R^2$ für einen Alkylrest mit 1 bis 8 C-Atomen steht, der durch ein Chlor-, ein Brom- oder ein Iodatom substituiert ist, das Halogenatom in üblicher Weise gegen eine Alkoxy- oder eine Cyangruppe austauscht.

Die hierbei als Ausgangssubstanzen verwendeten Verbindungen werden - wie oben angegeben - durch Behandlung einer Verbindung der Formel II mit einem Acylierungsmittel der Formel III, in dem $R^5$ für einen Alkylrest mit 1 bis 8 C-Atomen steht, der durch ein Chlor-, ein Brom- oder ein Iodatom substituiert ist, erhalten.

Der Austausch des Halogenatoms gegen einen Alkoxyrest kann durch Umsetzung mit einem Alkohol der Formel ROH, in der R einen Alkylrest mit 1 bis 4 C-Atomen bedeutet, in Gegenwart einer Hilfsbase als säurebindendes Mittel unter an sich üblichen Bedingungen durchgeführt werden, in der Regel unter Verwendung von wenigstens äquimolaren Mengen des Alkohols und der Hilfsbase, zweckmäßig in Gegenwart eines Lösungsmittels, bei einer ausreichenden Temperatur von im allgemeinen bis zu 80 °C, gegebenenfalls bei der Siedetemperatur des Reaktionsgemisches, und gegebenenfalls unter Anwendung von Druck.

Als Lösungsmittel kommen unter den Reaktionsbedingungen inerte Lösungsmittel, wie aromatische Kohlenwasserstoffe, beispielsweise Toluol oder Xylol, cyclische aliphatische Ether, zum Beispiel Tetrahydrofuran oder Dioxan, oder Dialkylformamide, insbesondere Dimethylformamid, in Betracht. Es kann aber auch in dem Alkohol als Lösungsmittel gearbeitet werden.

Hilfsbasen als säurebindende Mittel sind zweckmäßigerweise anorganische Basen, wie Natriumhydrid, Natrium- bzw. Kaliumcarbonat oder Natrium- bzw. Kaliumhydrogencarbonat, oder tertiäre organische Amine, wie Triethylamin.

Der Austausch des Halogenatoms gegen einen Alkoxyrest kann auch durch Umsetzung mit einem Natrium- oder Kaliumalkoholat der Formel RONa bzw. ROK, wobei R einen Alkylrest mit 1 bis 4 C-Atomen bedeutet, in an sich üblicher Weise durchgeführt werden, in der Regel unter Verwendung von wenigstens einer äquimolaren Menge des Alkoholats, in einem Lösungsmittel, und zwar in dem entsprechenden Alkohol oder in einem unter den Reaktionsbedingungen inerten Lösungsmittel, wie einem aromatischen Kohlenwasserstoff, zum Beispiel Toluol, einem cyclischen aliphatischen Ether, wie Tetrahydrofuran oder Dioxan, oder einem Dialkylformamid, insbesondere Dimethylformamid, bei Temperaturen von 0 bis 100 °C, gegebenenfalls bei der Siedetemperatur des Reaktionsgemisches, und gegebenenfalls unter Anwendung von Druck.

Der Austausch des Halogenatoms gegen eine Cyangruppe kann durch Behandlung mit Alkalicyanid, vorzugsweise Natrium- oder Kaliumcyanid, entweder in einem geeigneten Lösungsmittel, beispielsweise einem niederen Alkohol,

wie Methanol, Ethanol oder Propanol, oder einem Dialkylformamid, insbesondere Dimethylformamid, oder in einem Gemisch aus Wasser und einem der oben genannten niederen Alkohole oder in einem Zwei-Phasensystem bestehend aus Wasser und einem mit Wasser nicht mischbaren Lösungsmittel, wie einem aromatischen Kohlenwasserstoff, beispielsweise Toluol oder Xylol, einem aliphatischen oder aromatischen Chlorkohlenwasserstoff, zum Beispiel Methylenchlorid, Ethylenchlorid oder Chlorbenzol oder einem höheren aliphatischen Keton, beispielsweise Methylisobutylketon, bei Temperaturen zwischen 0 und 140 °C, vorzugsweise 20 bis 100 °C, gegebenenfalls bei der Siedetemperatur des Reaktionsgemisches, und gegebenenfalls unter Anwendung von Druck geschehen. Bei der zuletzt angegebenen Arbeitsweise, der sogenannten Phasen-Transfer-Methode, ist es zweckmäßig, einen entsprechenden Katalysator, zum Beispiel ein quartäres Ammoniumsalz, vorzugsweise ein Halogenid, wie Tetrabutylammoniumbromid, Tetrabutylammoniumiodid, Benzyltriethylammoniumbromid oder Methyltridecylammoniumchlorid (Aliquat $^{R}$ 336), oder ein quartäres Phosphoniumsalz, ebenfalls vorzugsweise ein Halogenid, zum Beispiel Tetrabutylphosphoniumbromid oder Ethyltrioctylphosphoniumbromid, zuzusetzen. Dieser Katalysator wird in der Regel in Mengen von 0,1 bis 10 Mol-% verwendet. Es können aber auch bis zu 1-molare Mengen des Katalysators eingesetzt werden.

Die neuen Verbindungen der Formel I, in denen $R^1$ für ein Wasserstoffatom und $R^2$ für ein Wasserstoffatom oder einen Cycloalkylrest mit 3 bis 8 C-Atomen im Ring, der gegebenenfalls ein- bis vierfach durch Alkylreste mit 1 bis 4 C-Atomen substituiert ist, stehen, können auch erhalten werden, indem man ein Anilid der Formel VI,

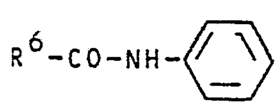

(VI)

in der $R^6$ ein Wasserstoffatom oder einen Cycloalkylrest mit 3 bis 8 C-Atomen im Ring, der gegebenenfalls ein- bis vierfach durch Alkylreste mit 1 bis 4 C-Atomen substituiert ist, bedeutet, mit Maleinsäureanhydrid in Gegenwart einer Lewis-Säure, bevorzugt Aluminiumchlorid, unter den Bedingungen einer Friedel-Crafts-Acylierung umsetzt und die dabei erhaltene Acrylsäure der Formel VII,

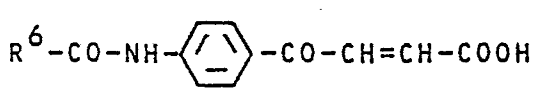

(VII)

in der $R^6$ die für Formel VI angegebenen Bedeutungen hat, mit Hydrazin cyclisiert.

Zur Friedel-Crafts-Acylierung eines Anilids der Formel VI mit Maleinsäureanhydrid kann beispielsweise unter den Bedingungen gearbeitet werden, die von D. Papa, E. Schwenk, F. Villani und E. Klingsberg im Journal of the American Chemical Society, Band 70 (1948), S. 3356 - 3360, für die Herstellung von 3-(p-Acetylaminobenzoyl)acrylsäure aus Acetanilid und Maleinsäureanhydrid angegeben werden.

Die Cyclisierung einer Acrylsäure der Formel VII mit Hydrazin kann zum Beispiel nach der Verfahrensweise erfolgen, die von F. G. Baddar, A. El-Habashi und A. K. Fateen im Journal of the Chemical Society (1965), S. 2 - 3348, für die Cyclisierung von 3-(p-Toluoyl)acrylsäure mit Hydrazin zum 6-(p-Tolyl)-3(2H)-pyridazinon beschrieben wird.

Eine weitere Möglichkeit zu den neuen Verbindungen der Formel I zu gelangen, in denen $R^1$ eine Methylgruppe und $R^2$ ein Wasserstoffatom, einen Alkylrest mit 2 bis 8 C-Atomen oder einen Cycloalkylrest mit 3 bis 8 C-Atomen im Ring, der gegebenenfalls ein- bis vierfach durch Alkylreste mit 1 bis 4 C-Atomen substituiert ist, bedeuten, besteht darin, daß man ein Anilid der Formel VIII,

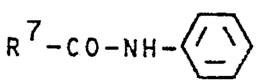
(VIII)

in der $R^7$ für ein Wasserstoffatom, einen Alkylrest mit 2 bis 8 C-Atomen oder einen Cycloalkylrest mit 3 bis 8 C-Atomen im Ring, der gegebenenfalls ein- bis vierfach durch Alkylreste mit 1 bis 4 C-Atomen substituiert ist, steht, mit Citraconsäureanhydrid in Gegenwart einer Lewis-Säure, bevorzugt Aluminiumchlorid, unter den Bedingungen einer Friedel-Crafts-Acylierung umsetzt und die dabei erhaltene Crotonsäure der Formel IX,

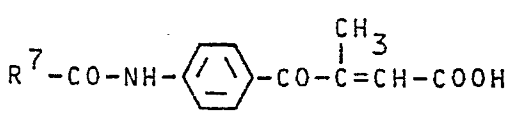
(IX)

in der $R^7$ die für Formel VIII angegebenen Bedeutungen hat, mit Hydrazin cyclisiert.

In der DE-OS 2 150 436 wird die Friedel-Crafts-Acylierung der Anilide VIII, in denen $R^7$ für ein Wasserstoffatom oder einen Alkylrest mit 2 bis 3 C-Atomen steht, mit Citraconsäureanhydrid zu den entsprechenden Crotonsäuren der Formel IX beschrieben. Beispielsweise unter den hierfür angegebenen Reaktionsbedingungen läßt sich auch die Friedel-Crafts-Acylierung der anderen Anilide VIII mit Citraconsäureanhydrid bewerkstelligen.

Zur Cyclisierung der Crotonsäuren der Formel IX mit Hydrazin kann zum Beispiel unter den Bedingungen gearbeitet werden, die von W. V. Curran und A. Ross im Journal of Medicinal Chemistry, Band 17 (1974), S. 273 bis 281, für die Herstellung von 6-(p-Acetylaminophenyl)-5-methyl-3(2H)--pyridazinon aus 3-(p-Acetylaminobenzoyl)-crotonsäure und Hydrazin angegeben werden.

Das als Ausgangssubstanz verwendete Aminophenyl-pyridazinon II mit $R^1$=H ist bekannt (siehe englische Patentschrift 1 168 291; E. A. Steck, R. P. Brundage und L. T. Fletcher, J. Heterocycl. Chem., 11, 755 - 761 (1974); DE-OS 2 810 267).

Aus einer Veröffentlichung von W. V. Curran und A. Ross (J. Med. Chem., 17, 273 - 281 (1974)) geht die Herstellung des als Ausgangssubstanz verwendeten Aminophenyl-pyridazinon II mit $R^1=CH_3$ hervor. Hergestellt wird diese Verbindung durch Cyclisierung der aus Acetanilid und Citraconsäureanhydrid zugänglichen 3-(p-Acetylaminobenzoyl)crotonsäure mit Hydrazin und Verseifung des dabei erhaltenen 6-(p-Acetylaminophenyl)-5-methyl--3(2H)-pyridazinons. Das Pyridazinon II mit $R^1=CH_3$ kann aber auch - wie wir fanden - durch Oxidation des entsprechenden 4,5-Dihydro-Derivats oder eines 6-(p-Alkanoylaminophenyl)-4,5-dihydro-5-methyl-3(2H)-pyrid azinons mit Natrium-m-nitrobenzolsulfonat in Gegenwart von Natronlauge synthetisiert werden. Diese Methode zur Herstellung von II mit $R^1=CH_3$ lehnt sich an ein von G. Bachmann beschriebenes Verfahren zur Herstellung von in den Stellungen 4 und 5 unsubstituierten 6-Aminophenyl-3(2H)-pyridazinonen an (siehe englische Patentschrift 1 168 291). Die benötigten 4,5-Dihydro-5--methyl-3(2H)-pyridazinone sind bekannt (siehe DE-OS 2 150 S-PS 3 824 271 und US-PS 3 888 901).

Zu den als Ausgangssubstanzen verwendeten Aminophenyl-pyridazinonen II, in denen $R^1$ einen Alkylrest mit 2 oder 3 C-Atomen bedeutet, kann man ebenfalls durch Oxidation der entsprechenden 6-(p-Aminophenyl)- und 6-(p-Alkanoylaminophenyl)-4,5-dihydro-3(2H)-pyridazinone mit Natrium-m-nitrobenzolsulfonat in Gegenwart von Natronlauge gelangen. Die benötigten Dihydropyridazinone sind bekannt (siehe US-PSen 3 824 271 und 3 888 901).

Erfindungsgemäße Verbindungen, die nach den genannten Verfahren erhalten werden, sind beispielsweise:

6-(p-Formylaminophenyl)-3(2H)-pyridazinon;
6-(p-Formylaminophenyl)-5-methyl-3(2H)-pyridazinon;
6-(p-Propionylaminophenyl)-5-methyl-3(2H)-pyridazinon;
6-(p-Butyrylaminophenyl)-5-methyl-3(2H)-pyridazinon;
6-(p-Isobutyrylaminophenyl)-5-methyl-3(2H)-pyridazinon;
6-(p-Valerylaminophenyl)-5-methyl-3(2H)-pyridazinon;
6-(p-Isovalerylaminophenyl)-5-methyl-3(2H)-pyridazinon;
6-[p-(2-Methylbutyrylamino)phenyl]-5-methyl-3(2H)-pyridazinon;
6-(p-Pivaloylaminophenyl)-5-methyl-3(2H)-pyridazinon;
6-(p-Chloracetylaminophenyl)-3(2H)-pyridazinon;
6-(p-Chloracetylaminophenyl)-5-methyl-3(2H)-pyridazinon;
6-(p-Bromacetylaminophenyl)-3(2H)-pyridazinon;
6-(p-Bromacetylaminophenyl)-5-methyl-3(2H)-pyridazinon;
6-(p-Fluoracetylaminophenyl)-3(2H)-pyridazinon;
6-(p-Fluoracetylaminophenyl)-5-methyl-3(2H)-pyridazinon;
6-(p-Iodacetylaminophenyl)-3(2H)-pyridazinon;
6-(p-Iodacetylaminophenyl)-5-methyl-3(2H)-pyridazinon;
6-[p-(2-Chlorpropionylamino)phenyl]-3(2H)-pyridazinon;
6-[p-(2-Chlorpropionylamino)phenyl]-5-methyl-3(2H)-pyridazinon;
6-[p-(2-Brompropionylamino)phenyl]-3(2H)-pyridazinon;
6-[p-(2-Brompropionylamino)phenyl]-5-methyl-3(2H)-pyridazinon;
6-[p-(2-Fluorpropionylamino)phenyl]-3(2H)-pyridazinon;
6-[p-(2-Fluorpropionylamino)phenyl]-5-methyl-3(2H)-pyridazinon;
6-[p-(3-Chlorpropionylamino)phenyl]-3(2H)-pyridazinon;
6-[p-(3-Chlorpropionylamino)phenyl]-5-methyl-3(2H)-pyridazinon;
6-[p-(3-Brompropionylamino)phenyl]-3(2H)-pyridazinon;
6-[p-(3-Brompropionylamino)phenyl]-5-methyl-3(2H)-pyridazinon;
6-[p-(3-Fluorpropionylamino)phenyl]-3(2H)-pyridazinon;
6-[p-(3-Fluorpropionylamino)phenyl]-5-methyl-3(2H)-pyridazinon;
6-[p-(2-Chlorbutyrylamino)phenyl]-3(2H)-pyridazinon;
6-[p-(2-Chlorbutyrylamino)phenyl]-5-methyl-3(2H)-pyridazinon;
6-[p-(2-Brombutyrylamino)phenyl]-3(2H)-pyridazinon;
6-[p-(2-Brombutyrylamino)phenyl]-5-methyl-3(2H)-pyridazinon;
6-[p-(4-Chlorbutyrylamino)phenyl]-3(2H)-pyridazinon;
6-[p-(2-Chlorisobutyrylamino)phenyl]-3(2H)-pyridazinon;
6-[p-(2-Chlorisobutyrylamino)phenyl]-5-methyl-3(2H)-pyridazinon;
6-[p-(3-Chlorisobutyrylamino)phenyl]-3(2H)-pyridazinon;
6-[p-(2-Bromvalerylamino)phenyl]-3(2H)-pyridazinon;
6-(p-Chlorpivaloylaminophenyl)-3(2H)-pyridazinon;
6-(p-Chlorpivaloylaminophenyl)-5-methyl-3(2H)-pyridazinon;

6-(p-Brompivaloylaminophenyl)-3(2H)-pyridazinon;
6-[p-(2-Brom-2-ethylbutyrylamino)phenyl]-3(2H)-pyridazinon;

6-(p-Dichloracetylaminophenyl)-3(2H)-pyridazinon;
6-(p-Dichloracetylaminophenyl)-5-methyl-3(2H)-pyridazinon;
6-(p-Difluoracetylaminophenyl)-3(2H)-pyridazinon;
6-(p-Difluoracetylaminophenyl)-5-methyl-3(2H)-pyridazinon;
6-[p-(2,2-Dichlorpropionylamino)phenyl]-3(2H)-pyridazinon;
6-[p-(2,2-Dichlorpropionylamino)phenyl]-5-methyl-3(2H)-pyridazinon;
6-[p-(2,3-Dichlorpropionylamino)phenyl]-3(2H)-pyridazinon;
6-[p-(2,3-Dichlorpropionylamino)phenyl]-5-methyl-3(2H)-pyridazinon;
6-[p-(2,2-Dichlorbutyrylamino)phenyl]-3(2H)-pyridazinon;
6-[p-(2,2-Dichlorbutyrylamino)phenyl]-5-methyl-3(2H)-pyridazinon;
6-[p-(2,4-Dichlorbutyrylamino)phenyl]-3(2H)-pyridazinon;
6-(p-Trichloracetylaminophenyl)-3(2H)-pyridazinon;
6-(p-Trifluoracetylaminophenyl)-3(2H)-pyridazinon;
6-(p-Trifluoracetylaminophenyl)-5-methyl-3(2H)-pyridazinon;
6-(p-Chlordifluoracetylaminophenyl)-3(2H)-pyridazinon;
6-(p-Chlordifluoracetylaminophenyl)-5-methyl-3(2H)-pyridazinon;
6-(p-Hydroxyacetylaminophenyl)-3(2H)-pyridazinon;
6-(p-Hydroxyacetylaminophenyl)-5-methyl-3(2H)-pyridazinon;
6-[p-(2-Hydroxypropionylamino)phenyl]-3(2H)-pyridazinon;
6-[p-(2-Hydroxypropionylamino)phenyl]-5-methyl-3(2H)-pyridazinon;
6-[p-(3-Hydroxypropionylamino)phenyl]-3(2H)-pyridazinon;
6-[p-(3-Hydroxypropionylamino)phenyl]-5-methyl-3(2H)-pyridazinon;
6-[p-(2-Hydroxybutyrylamino)phenyl]-3(2H)-pyridazinon;
6-[p-(2-Hydroxybutyrylamino)phenyl]-5-methyl-3(2H)-pyridazinon;
6-[p-(3-Hydroxybutyrylamino)phenyl]-3(2H)-pyridazinon;
6-[p-(4-Hydroxybutyrylamino)phenyl]-3(2H)-pyridazinon;
6-[p-(2-Hydroxyisobutyrylamino)phenyl]-3(2H)-pyridazinon;
6-[p-(2-Hydroxyvalerylamino)phenyl]-3(2H)-pyridazinon;

6-(p-Methoxyacetylaminophenyl)-3(2H)-pyridazinon;
6-(p-Methoxyacetylaminophenyl)-5-methyl-3(2H)-pyridazinon;
6-(p-Ethoxyacetylaminophenyl)-3(2H)-pyridazinon;
6-(p-Ethoxyacetylaminophenyl)-5-methyl-3(2H)-pyridazinon;
6-[p-(2-Methoxypropionylamino)phenyl]-3(2H)-pyridazinon;
6-[p-(2-Methoxypropionylamino)phenyl]-5-methyl-3(2H)-pyridazinon;
6-[p-(2-Ethoxypropionylamino)phenyl]-3(2H)-pyridazinon;
6-[p-(2-Ethoxypropionylamino)phenyl]-5-methyl-3(2H)-pyridazinon;
6-[p-(3-Methoxypropionylamino)phenyl]-3(2H)-pyridazinon;
6-[p-(3-Methoxypropionylamino)phenyl]-5-methyl-3(2H)-pyridazinon;
6-[p-(3-Ethoxypropionylamino)phenyl]-3(2H))-pyridazinon;

6-[p-(3-Ethoxypropionylamino)phenyl]-5-methyl-3(2H)-pyridazinon;
6-[p-(2-Methoxybutyrylamino)phenyl]-3(2H)-pyridazinon;
6-[p-(2-Methoxybutyrylamino)phenyl]-5-methyl-3(2H)-pyridazinon;
6-[p-(2-Ethoxybutyrylamino)phenyl]-3(2H)-pyridazinon;
6-[p-(3-Methoxybutyrylamino)phenyl]-3(2H)-pyridazinon;
6-[p-(3-Methoxybutyrylamino)phenyl]-5-methyl-3(2H)-pyridazinon;
6-[p-(3-Ethoxybutyrylamino)phenyl]-3(2H)-pyridazinon;
6-[p-(4-Methoxybutyrylamino)phenyl]-3(2H)-pyridazinon;
6-[p-(2-Methoxyisobutyrylamino)phenyl]-3(2H)-pyridazinon;
6-[p-(3-Methoxyisobutyrylamino)phenyl]-3(2H)-pyridazinon;
6-[p-(2-Methoxyvalerylamino)phenyl]-3(2H)-pyridazinon;

6-(p-Acetoxyacetylaminophenyl)-3(2H)-pyridazinon;
6-(p-Acetoxyacetylaminophenyl)-5-methyl-3(2H)-pyridazinon;
6-(p-Propionyloxyacetylaminophenyl)-3(2H)-pyridazinon;
6-(p-Propionyloxyacetylaminophenyl)-5-methyl-3(2H)-pyridazinon;
6-[p-(2-Acetoxypropionylamino)phenyl]-3(2H)-pyridazinon;
6-[p-(2-Acetoxypropionylamino)phenyl]-5-methyl-3(2H)-pyridazinon;
6-[p-(2-Propionyloxypropionylamino)phenyl]-3(2H)-pyridazinon;
6-[p-(3-Acetoxypropionylamino)phenyl]-3(2H)-pyridazinon;
6-[p-(3-Acetoxypropionylamino)phenyl]-5-methyl-3(2H)-pyridazinon;
6-[p-(3-Propionyloxypropionylamino)phenyl]-3(2H)-pyridazinon;
6-[p-(2-Acetoxybutyrylamino)phenyl]-3(2H)-pyridazinon;
6-[p-(2-Acetoxybutyrylamino)phenyl]-5-methyl-3(2H)-pyridazinon;
6-[p-(2-Propionyloxybutyrylamino)phenyl]-3(2H)-pyridazinon;
6-[p-(3-Acetoxybutyrylamino)phenyl]-3(2H)-pyridazinon;
6-[p-(3-Acetoxybutyrylamino)phenyl]-5-methyl-3(2H)-pyridazinon;
6-[p-(4-Acetoxybutyrylamino)phenyl]-3(2H)-pyridazinon;
6-[p-(2-Acetoxyisobutyrylamino)phenyl]-3(2H)-pyridazinon;
6-[p-(2-Acetoxyvalerylamino)phenyl]-3(2H)-pyridazinon;

6-(p-Cyanacetylaminophenyl)-3(2H)-pyridazinon;
6-(p-Cyanacetylaminophenyl)-5-methyl-3(2H)-pyridazinon;
6-[p-(2-Cyanpropionylamino)phenyl]-3(2H)-pyridazinon;
6-[p-(2-Cyanpropionylamino)phenyl]-5-methyl-3(2H)-pyridazinon;
6-[p-(3-Cyanpropionylamino)phenyl]-3(2H)-pyridazinon;
6-[p-(3-Cyanpropionylamino)phenyl]-5-methyl-3(2H)-pyridazinon;
6-[p-(2-Cyanbutyrylamino)phenyl]-3(2H)-pyridazinon;
6-[p-(2-Cyanbutyrylamino)phenyl]-5-methyl-3(2H)-pyridazinon;
6-[p-(3-Cyanbutyrylamino)phenyl]-3(2H)-pyridazinon;
6-[p-(4-Cyanbutyrylamino)phenyl]-3(2H)-pyridazinon;
6-[p-(2-Cyanisobutyrylamino)phenyl]-3(2H)-pyridazinon;
6-[p-(2-Cyanisobutyrylamino)phenyl]-5-methyl-3(2H)-pyridazinon;

6-[p-(3-Cyanisobutyrylamino)phenyl]-3(2H)-pyridazinon;
6-[p-(2-Cyanvalerylamino)phenyl]-3(2H)-pyridazinon;

6-(p-Cyclopropylcarbonylaminophenyl)-3(2H)-pyridazinon;
6-(p-Cyclopropylcarbonylaminophenyl)-5-methyl-3(2H)-pyridazinon;
6-[p-(1-Methylcyclopropylcarbonylamino)phenyl]-3(2H)-pyridazinon;
6-[p-(1-Methylcyclopropylcarbonylamino)phenyl]-5-methyl-3(2H)-pyridazinon;
cis- und trans-6-[p-(2-Methylcyclopropylcarbonylamino)phenyl]-3(2H)--pyridazinon;
cis- und trans-6-[p-(2-Methylcyclopropylcarbonylamino)phenyl]-5-methyl--3(2H)-pyridazinon;
6-[p-(2,2-Dimethylcyclopropylcarbonylamino)phenyl]-3(2H)-pyridazinon;
6-[p-(2,2-Dimethylcyclopropylcarbonylamino)phenyl]-5-methyl-3(2H)--pyridazinon;
6-[p-(1-Chlorcyclopropylcarbonylamino)phenyl]-3(2H)-pyridazinon;
6-[p-(1-Chlorcyclopropylcarbonylamino)phenyl]-5-methyl-3(2H)-pyridazinon;
cis- und trans-6-[p-(2-Bromcyclopropylcarbonylamino)phenyl]-3(2H)--pyridazinon;
cis- und trans-6-[p-(2-Bromcyclopropylcarbonylamino)phenyl]-5-methyl--3(2H)-pyridazinon;
6-[p-(2,2-Dichlorcyclopropylcarbonylamino)phenyl]-3(2H)-pyridazinon;
6-[p-(2,2-Dichlor-1-methylcyclopropylcarbonylamino)phenyl]-3(2H)-pyridazinon;
6-(p-Cyclobutylcarbonylaminophenyl)-3(2H)-pyridazinon;
6-(p-Cyclobutylcarbonylaminophenyl)-5-methyl-3(2H)-pyridazinon;
6-[p-(1-Methylcyclobutylcarbonylamino)phenyl]-3(2H)-pyridazinon;
cis- und trans-6-[p-(2-Methylcyclobutylcarbonylamino)phenyl]--3(2H)-pyridazinon;
6-[p-(1-Chlorcyclobutylcarbonylamino)phenyl]-3(2H)-pyridazinon;
6-[p-(1-Chlorcyclobutylcarbonylamino)phenyl]-5-methyl-3(2H)-pyridazinon;
cis- und trans-6-[p-(3-Chlorcyclobutylcarbonylamino)phenyl]-3(2H)-pyridazinon;
6-(p-Cyclopentylcarbonylaminophenyl)-3(2H)-pyridazinon;
6-(p-Cyclopentylcarbonylaminophenyl)-5-methyl-3(2H)-pyridazinon;
6-[p-(1-Methylcyclopentylcarbonylamino)phenyl]-3(2H)-pyridazinon;
6-[p-(1-Chlorcyclopentylcarbonylamino)phenyl]-3(2H)-pyridazinon;
6-(p-Cyclohexylcarbonylaminophenyl)-3(2H)-pyridazinon;
6-(p-Cyclohexylcarbonylaminophenyl)-5-methyl-3(2H)-pyridazinon;

6-(p-Acryloylaminophenyl)-3(2H)-pyridazinon;
6-(p-Acryloylaminophenyl)-5-methyl-3(2H)-pyridazinon;
6-(p-Crotonoylaminophenyl)-3(2H)-pyridazinon;
6-(p-Crotonoylaminophenyl)-5-methyl-3(2H)-pyridazinon;

6-(p-Methacryloylaminophenyl)-3(2H)-pyridazinon;
6-(p-Methacryloylaminophenyl)-5-methyl-3(2H)-pyridazinon;
6-[p-(But-3-enoylamino)phenyl]-3(2H)-pyridazinon;
6-[p-(But-3-enoylamino)phenyl]-5-methyl-3(2H)-pyridazinon;
6-[p-(3-Methylbut-2-enoylamino)phenyl]-3(2H)-pyridazinon;

6-(p-Methoxycarbonylaminophenyl)-3(2H)-pyridazinon;
6-(p-Methoxycarbonylaminophenyl)-5-methyl-3(2H)-pyridazinon;
6-(p-Ethoxycarbonylaminophenyl)-3(2H)-pyridazinon;
6-(p-Ethoxycarbonylaminophenyl)-5-methyl-3(2H)-pyridazinon;
6-(p-Propoxycarbonylaminophenyl)-3(2H)-pyridazinon;
6-(p-Propoxycarbonylaminophenyl)-5-methyl-3(2H)-pyridazinon;
6-(p-Isopropoxycarbonylaminophenyl)-3(2H)-pyridazinon;
6-(p-Isopropoxycarbonylaminophenyl)-5-methyl-3(2H)-pyridazinon;
6-(p-Butoxycarbonylaminophenyl)-3(2H)-pyridazinon;
6-(p-Butoxycarbonylaminophenyl)-5-methyl-3(2H)-pyridazinon;
6-(p-Isobutoxycarbonylaminophenyl)-3(2H)-pyridazinon;
6-[p-(sec-Butoxy)carbonylaminophenyl]-3(2H)-pyridazinon;
6-[p-(tert-Butoxy)carbonylaminophenyl]-3(2H)-pyridazinon;

6-(p-Chlormethoxycarbonylaminophenyl)-3(2H)-pyridazinon;
6-(p-Chlormethoxycarbonylaminophenyl)-5-methyl-3(2H)-pyridazinon;
6-(p-Dichlormethoxycarbonylaminophenyl)-3(2H)-pyridazinon;
6-(p-Dichlormethoxycarbonylaminophenyl)-5-methyl-3(2H)-pyridazinon;
6-(p-Trifluormethoxycarbonylaminophenyl)-3(2H)-pyridazinon;
6-[p-(1-Chlorethoxycarbonylamino)phenyl]-3(2H)-pyridazinon;
6-[p-(1-Chlorethoxycarbonylamino)phenyl]-5-methyl-3(2H)-pyridazinon;
6-[p-(2-Chlorethoxycarbonylamino)phenyl]-3(2H)-pyridazinon;
6-[p-(2-Chlorethoxycarbonylamino)phenyl]-5-methyl-3(2H)-pyridazinon;
6-[p-(2-Bromethoxycarbonylamino)phenyl]-3(2H)-pyridazinon;
6-[p-(2-Bromethoxycarbonylamino)phenyl]-5-methyl-3(2H)-pyridazinon;
6-[p-(2-Fluorethoxycarbonylamino)phenyl]-3(2H)-pyridazinon;
6-[p-(2-Fluorethoxycarbonylamino)phenyl]-5-methyl-3(2H)-pyridazinon;
6-[p-(1,2-Dichlorethoxycarbonylamino)phenyl]-3(2H)-pyridazinon;
6-[p-(1,2-Dichlorethoxycarbonylamino)phenyl]-5-methyl-3(2H)-pyridazinon;
6-[p-(2,2,2-Trichlorethoxycarbonylamino)phenyl]-3(2H)-pyridazinon;
6-[p-(2,2,2-Trifluorethoxycarbonylamino)phenyl]-3(2H)-pyridazinon;
6-[p-(2,2,2-Trifluorethoxycarbonylamino)phenyl]-5-methyl-3(2H)-pyridazinon;
6-[p-(2-Brompropoxycarbonylamino)phenyl]-3(2H)-pyridazinon;
6-[p-(3-Chlorpropoxycarbonylamino)phenyl]-3(2H)-pyridazinon;
6-[p-(2,3-Dichlorpropoxycarbonylamino)phenyl]-3(2H)-pyridazinon;
6-[p-(2-Chlorisopropoxycarbonylamino)phenyl]-3(2H)-pyridazinon;
6-[p-(2-Chlorbutoxycarbonylamino)phenyl]-3(2H)-pyridazinon;

6-[p-(2-Chlorbutoxycarbonylamino)phenyl]-5-methyl-3(2H)-pyridazinon;
6-[p-(1-Chlormethylpropoxycarbonylamino)phenyl]-3(2H)-pyridazinon;
6-[p-(1-Chlormethylpropoxycarbonylamino)phenyl]-5-methyl-3(2H)-pyridazinon;

6-[p-(2-Methoxyethoxycarbonylamino)phenyl]-3(2H)-pyridazinon;
6-[p-(2-Methoxyethoxycarbonylamino)phenyl]-5-methyl-3(2H)-pyridazinon;
6-[p-(2-Ethoxyethoxycarbonylamino)phenyl]-3(2H)-pyridazinon;
6-[p-(2-Methoxypropoxycarbonylamino)phenyl]-3(2H)-pyridazinon;
6-[p-(2-Ethoxypropoxycarbonylamino)phenyl]-3(2H)-pyridazinon;
6-[p-(3-Methoxypropoxycarbonylamino)phenyl]-3(2H)-pyridazinon;
6-[p-(3-Ethoxypropoxycarbonylamino)phenyl]-3(2H)-pyridazinon;
6-[p-(2-Methoxyisopropoxycarbonylamino)phenyl]-3(2H)-pyridazinon;

6-(p-Benzyloxycarbonylaminophenyl)-3(2H)-pyridazinon;
6-(p-Benzyloxycarbonylaminophenyl)-5-methyl-3(2H)-pyridazinon;
6-[p-(1-Phenylethoxycarbonylamino)phenyl]-3(2H)-pyridazinon;
6-[p-(1-Phenylethoxycarbonylamino)phenyl]-5-methyl-3(2H)-pyridazinon;
6-[p-(2-Phenylethoxycarbonylamino)phenyl]-3(2H)-pyridazinon;
6-[p-(1-Phenylpropoxycarbonylamino)phenyl]-3(2H)-pyridazinon;
6-[p-(2-Phenylpropoxycarbonylamino)phenyl]-3(2H)-pyridazinon;
6-[p-(1-Methyl-2-phenylethoxycarbonylamino)phenyl]-3(2H)-pyridazinon;
6-[p-(p-Methylbenzyloxycarbonylamino)phenyl]-3(2H)-pyridazinon;
6-[p-(m-Methoxybenzyloxycarbonylamino)phenyl]-3(2H)-pyridazinon;
6-[p-(o-Chlorbenzyloxycarbonylamino)phenyl]-3(2H)-pyridazinon;
6-[p-(m-Chlorbenzyloxycarbonylamino)phenyl]-3(2H)-pyridazinon;
6-[p-(p-Chlorbenzyloxycarbonylamino)phenyl]-3(2H)-pyridazinon;
6-[p-(p-Fluorbenzyloxycarbonylamino)phenyl]-3(2H)-pyridazinon;
6-[p-(m-Trifluormethylbenzyloxycarbonylamino)phenyl]-3(2H)-pyridazinon;
6-[p-(p-Nitrobenzyloxycarbonylamino)phenyl]-3(2H)-pyridazinon;
6-[p-[2-(m,p-Dimethoxyphenyl)ethoxycarbonylamino]phenyl]-3(2H)-pyridazinon;

6-(p-Cyclopropyloxycarbonylaminophenyl)-3(2H)-pyridazinon;
6-(p-Cyclopropyloxycarbonylaminophenyl)-5-methyl-3(2H)-pyridazinon;
6-[p-(1-Methylcyclopropyloxycarbonylamino)phenyl]-3(2H)-pyridazinon;
6-[p-(2-Methylcyclopropyloxycarbonylamino)phenyl]-3(2H)[-pyridazinon;
6-[p-(2,2-Dimethylcyclopropyloxycarbonylamino)phenyl]-3(2H)-pyridazinon;
6-(p-Cyclobutyloxycarbonylaminophenyl)-3(2H)-pyridazinon;
6-(p-Cyclobutyloxycarbonylaminophenyl)-5-methyl-3(2H)-pyridazinon;
6-[p-(1-Methylcyclobutyloxycarbonylamino)phenyl]-3(2H)-pyridazinon;
6-[p-(2-Methylcyclobutyloxycarbonylamino)phenyl]-3(2H)-pyridazinon;
6-[p-(3-Methylcyclobutyloxycarbonylamino)phenyl]-3(2H)-pyridazinon;
6-[p-(2,2-Dimethylcyclobutyloxycarbonylamino)phenyl]-3(2H)-pyridazinon;

6-[p-(3,3-Dimethylcyclobutyloxycarbonylamino)phenyl]-3(2H)-pyridazinon;
6-(p-Cyclopentyloxycarbonylaminophenyl)-3(2H)-pyridazinon;
6-(p-Cyclohexyloxycarbonylaminophenyl)-3(2H)-pyridazinon;

6-(p-Allyloxycarbonylaminophenyl)-3(2H)-pyridazinon;
6-(p-Allyloxycarbonylaminophenyl)-5-methyl-3(2H)-pyridazinon;
6-[p-(But-2-enyloxycarbonylamino)phenyl]-3(2H)-pyridazinon;
6-[p-(But-3-enyloxycarbonylamino)phenyl]-3(2H)-pyridazinon;
6-[p-(1-Methylallyloxycarbonylamino)phenyl]-3(2H)-pyridazinon;
6-[p-(2-Methylallyloxycarbonylamino)phenyl]-3(2H)-pyridazinon;

6-[p-(Prop-2-inyloxycarbonylamino)phenyl]-3(2H)-pyridazinon;
6-[p-(Prop-2-inyloxycarbonylamino)phenyl]-5-methyl-3(2H)-pyridazinon;
6-[p-(But-2-inyloxycarbonylamino)phenyl]-3(2H)-pyridazinon;
6-[p-(But-2-inyloxycarbonylamino)phenyl]-5-methyl-3(2H)-pyridazinon;
6-[p-(But-3-inyloxycarbonylamino)phenyl]-3(2H)-pyridazinon;
6-[p-(But-3-inyloxycarbonylamino)phenyl]-5-methyl-3(2H)-pyridazinon;
6-[p-(1-Methylprop-2-inyloxycarbonylamino)phenyl]-3(2H)-pyridazinon;
6-[p-(1-Methylprop-2-inyloxycarbonylamino)phenyl]-5-methyl-3(2H)-pyridazinon;
6-[p-(Pent-2-inyloxycarbonylamino)phenyl]-3(2H)-pyridazinon;
6-[p-(1,1-Dimethylprop-2-inyloxycarbonylamino)phenyl]-3(2H)-pyridazinon;

6-(p-Phenoxycarbonylaminophenyl)-3(2H)-pyridazinon;
6-(p-Phenoxycarbonylaminophenyl)-5-methyl-3(2H)-pyridazinon;
6-[p-(m-Tolyloxycarbonylamino)phenyl]-3(2H)-pyridazinon;
6-[p-(o-Methoxyphenoxycarbonylamino)phenyl]-3(2H)-pyridazinon;
6-[p-(m-Methoxyphenoxycarbonylamino)phenyl]-3(2H)-pyridazinon;
6-[p-(o-Chlorphenoxycarbonylamino)phenyl]-3(2H)-pyridazinon;
6-[p-(m-Chlorphenoxycarbonylamino)phenyl]-3(2H)-pyridazinon;
6-[p-(p-Chlorphenoxycarbonylamino)phenyl]-3(2H)-pyridazinon;
6-[p-(p-Fluorphenoxycarbonylamino)phenyl]-3(2H)-pyridazinon;
6-[p-(m-Trifluormethylphenoxycarbonylamino)phenyl]-3(2H)-pyridazinon;
6-[p-(p-Cyanphenoxycarbonylamino)phenyl]-3(2H)-pyridazinon;
6-[p-(o-Nitrophenoxycarbonylamino)phenyl]-3(2H)-pyridazinon;
6-[p-(p-Nitrophenoxycarbonylamino)phenyl]-3(2H)-pyridazinon;

6-(p-Methylmercaptocarbonylaminophenyl)-3(2H)-pyridazinon;
6-(p-Methylmercaptocarbonylaminophenyl)-5-methyl-3(2H)-pyridazinon;
6-(p-Ethylmercaptocarbonylaminophenyl)-3(2H)-pyridazinon;
6-(p-Ethylmercaptocarbonylaminophenyl)-5-methyl-3(2H)-pyridazinon;
6-(p-Propylmercaptocarbonylaminophenyl)-3(2H)-pyridazinon;
6-(p-Propylmercaptocarbonylaminophenyl)-5-methyl-3(2H)-pyridazinon;

0116853

6-(p-Isopropylmercaptocarbonylaminophenyl)-3(2H)-pyridazinon;
6-(p-Isopropylmercaptocarbonylaminophenyl)-5-methyl-3(2H)-pyridazinon;
6-(p-Butylmercaptocarbonylaminophenyl)-3(2H)-pyridazinon;

6-(p-Trifluormethylmercaptocarbonylaminophenyl)-3(2H)-pyridazinon;
6-[p-(2-Chlorethylmercaptocarbonylamino)phenyl]-3(2H)-pyridazinon;
6-[p-(2-Chlorethylmercaptocarbonylamino)phenyl]-5-methyl-3(2H)-pyridazinon;
6-[p-(2-Bromethylmercaptocarbonylamino)phenyl]-3(2H)-pyridazinon;
6-[p-(2-Fluorethylmercaptocarbonylamino)phenyl]-3(2H)-pyridazinon;
6-[p-(2,2,2-Trifluorethylmercaptocarbonylamino)phenyl]-3(2H)-pyridazinon;
6-[p-(3-Chlorpropylmercaptocarbonylamino)phenyl]-3(2H)-pyridazinon;
6-[p-(2,3-Dichlorpropylmercaptocarbonylamino)phenyl]-3(2H)-pyridazinon;

6-[p-(2-Methoxyethylmercaptocarbonylamino)phenyl]-3(2H)-pyridazinon;
6-[p-(2-Ethoxyethylmercaptocarbonylamino)phenyl]-3(2H)-pyridazinon;

6-(p-Benzylmercaptocarbonylaminophenyl)-3(2H)-pyridazinon;
6-(p-Benzylmercaptocarbonylaminophenyl)-5-methyl-3(2H)-pyridazinon;
6-[p-(1-Phenylethylmercaptocarbonylamino)phenyl]-3(2H)-pyridazinon;
6-[p-(2-Phenylethylmercaptocarbonylamino)phenyl]-3(2H)-pyridazinon;
6-[p-(p-Methylbenzylmercaptocarbonylamino)phenyl]-3(2H)-pyridazinon;
6-[p-(p-Methoxybenzylmercaptocarbonylamino)phenyl]-3(2H)-pyridazinon;
6-[p-(p-Chlorbenzylmercaptocarbonylamino)phenyl]-3(2H)-pyridazinon;
6-[p-(p-Fluorbenzylmercaptocarbonylamino)phenyl]-3(2H)-pyridazinon;
6-[p-(m-Trifluormethylbenzylmercaptocarbonylamino)phenyl]-3(2H)-pyridazinon;
6-[p-(p-Nitrobenzylmercaptocarbonylamino)phenyl]-3(2H)-pyridazinon;

6-(p-Cyclopropylmercaptocarbonylaminophenyl)-3(2H)-pyridazinon;
6-(p-Cyclopropylmercaptocarbonylaminophenyl)-5-methyl-3(2H)-pyridazinon;
6-(p-Cyclobutylmercaptocarbonylaminophenyl)-3(2H)-pyridazinon;
6-(p-Cyclohexylmercaptocarbonylaminophenyl)-3(2H)-pyridazinon;

6-(p-Allylmercaptocarbonylaminophenyl)-3(2H)-pyridazinon;
6-(p-Allylmercaptocarbonylaminophenyl)-5-methyl-3(2H)-pyridazinon;
6-[p-(But-2-enylmercaptocarbonylamino)phenyl]-3(2H)-pyridazinon;
6-[p-(But-3-enylmercaptocarbonylamino)phenyl]-3(2H)-pyridazinon;

6-[p-(Prop-2-inylmercaptocarbonylamino)phenyl]-3(2H)-pyridazinon;
6-[p-(Prop-2-inylmercaptocarbonylamino)phenyl]-5-methyl-3(2H)-pyridazinon;

6-(p-Phenylmercaptocarbonylaminophenyl)-3(2H)-pyridazinon;
6-[p-(p-Tolylmercaptocarbonylamino)phenyl]-3(2H)-pyridazinon;

6-[p-(o-Methoxyphenylmercaptocarbonylamino)phenyl]-3(2H)-pyridazinon;
6-[p-(p-Chlorphenylmercaptocarbonylamino)phenyl]-3(2H)-pyridazinon;

6-[p-(p-Fluorphenylmercaptocarbonylamino)phenyl]-3(2H)-pyridazinon;
6-[p-(m-Trifluormethylphenylmercaptocarbonylamino)phenyl]-3(2H)-pyridazinon;
6-[p-(p-Cyanphenylmercaptocarbonylamino)phenyl]-3(2H)-pyridazinon;
6-[p-(p-Nitrophenylmercaptocarbonylamino)phenyl]-3(2H)-pyridazinon;

6-[p-(3-Methylureido)phenyl]-3(2H)-pyridazinon;
6-[p-(3-Methylureido)phenyl]-5-methyl-3(2H)-pyridazinon;
6-[p-(3-Ethylureido)phenyl]-3(2H)-pyridazinon;
6-[p-(3-Ethylureido)phenyl]-5-methyl-3(2H)-pyridazinon;
6-[p-(3-Propylureido)phenyl]-3(2H)-pyridazinon;
6-[p-(3-Propylureido)phenyl]-5-methyl-3(2H)-pyridazinon;
6-[p-(3-Isopropylureido)phenyl]-3(2H)-pyridazinon;
6-[p-(3-Isopropylureido)phenyl]-5-methyl-3(2H)-pyridazinon;
6-[p-(3-Butylureido)phenyl]-3(2H)-pyridazinon;
6-[p-(3-Butylureido)phenyl]-5-methyl-3(2H)-pyridazinon;
6-[p-(3-Isobutylureido)phenyl]-3(2H)-pyridazinon;
6-[p-[3-(sec-Butyl)ureido]phenyl]-3(2H)-pyridazinon;
6-[p-[3-(tert-Butyl)ureido]phenyl]-3(2H)-pyridazinon und
6-[p-(3-Pentylureido)phenyl]-3(2H)-pyridazinon.

Es wird darauf hingewiesen, daß in den Verbindungen der Formel I bei geeignetem Rest $R^2$, zum Beispiel 2-Methylcyclopropyl, eine geometrische (cis-trans) Isomerie auftritt. Die vorliegende Erfindung betrifft die jeweiligen cis- und trans-Isomeren und deren Gemische. Die reinen cis- oder trans-Verbindungen werden erhalten, indem man reine Ausgangsverbindungen verwendet, oder, indem man Gemische der cis- und trans-Verbindungen z. B. durch Kristallisation trennt.

Die erfindungsgemäßen Pyridazinone der Formel I zeichnen sich durch thrombozytenaggregationshemmende, blutdrucksenkende und magensekretionshemmende Eigenschaften aus. Sie sind als Antihypertensiva und zur Prophylaxe und Therapie thrombotischer Erkrankungen sowie zur Behandlung von Krankheitszuständen, die mit einer pathologisch gesteigerten Magensekretion einhergehen, z.B. Magen- und Duodenalulcera, geeignet.

Gegenstand der vorliegenden Erfindung sind demnach auch therapeutische Mittel oder Zubereitungen, die neben pharmazeutisch üblichen Träger- und Verdünnungsmitteln eine Verbindung der Formel I als Wirkstoff enthalten, sowie die Verwendung dieser Verbindungen zu therapeutischen Zwecken.

Die therapeutischen Mittel oder Zubereitungen werden mit den üblichen Trägerstoffen oder Verdünnungsmitteln und den üblicherweise verwendeten pharmazeutisch technischen Hilfsstoffen entsprechend der gewünschten Applikationsart mit einer geeigneten Dosierung in bekannter Weise hergestellt. Dabei kommen beim Menschen Dosen von 1 bis 100 mg in Betracht, wobei die orale Applikation bevorzugt ist.

Darreichungsformen, die zur oralen Applikation geeignet sind, sind beispielsweise Tabletten, Filmtabletten, Dragees, Kapseln, Pillen, Pulver, Lösungen, Suspensionen oder Depotformen.

Zur praktischen Anwendung werden die erfindungsgemäß zu verwendenden Verbindungen mit den in der galenischen Pharmazie üblichen Trägerstoffen verarbeitet. Die entsprechenden Tabletten können beispielsweise durch Mischen des Wirkstoffs mit bekannten Hilfsstoffen, beispielsweise inerten Verdünnungsmitteln, wie Dextrose, Zucker, Sorbit, Polyvinylpyrrolidon, Mannit, Calciumcarbonat, Calciumphosphat oder Milchzucker, Sprengmitteln, wie Mais, Stärke, Alginsäure oder Polyvinylpyrrolidon, Bindemitteln, wie Stärke oder Gelatine, Gleitmitteln, wie Magnesiumstearat oder Talkum und/oder Mitteln zur Erzielung des Depoteffektes, wie Carboxypolymethylen, Carboxymethylcellulose, Celluloseacetatphthalat oder Polyvinylacetat, erhalten werden. Die Tabletten können auch aus mehreren Schichten bestehen (vgl. H. Sucker et al., Pharmazeutische Technol., Thieme-Verlag, Stuttgart 1978).

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Im folgenden wird die Herstellung des als Ausgangssubstanz verwendeten Amino-phenyl-pyridazinons II mit $R^1 = CH_3$ beschrieben. Die Beispiele beschreiben die Herstellung erfindungsgemäßer Verbindungen.

Herstellung der Ausgangssubstanz II ($R^1 = CH_3$)

Ein Gemisch aus 50,7 g (249 mmol) 6-(p-Aminophenyl)-4,5-dihydro-5-methyl-3(2H)-pyridazinon, 28,1 g (125 mmol) Natrium-m-nitrobenzolsulfonat, 14,1 g (352 mmol) Natriumhydroxid und 280 ml Wasser wird unter Rühren 4 h bei Rückflußtemperatur gehalten. Das Reaktionsgemisch wird bei 20 °C filtriert und das Filtrat mit konzentrierter Salzsäure auf pH 6 eingestellt.

Die dabei erhaltene Festsubstanz wird abgesaugt, mit Wasser gewaschen und anschließend mit 14,0 g (62,2 mmol) Natrium-m-nitrobenzolsulfonat und 7,0 g (175 mmol) Natriumhydroxid in 280 ml Wasser unter Rühren 6 h bei Rückflußtemperatur gehalten. Die nach dem Filtrieren bei 20 °C, dem Einstellen des Filtrats auf pH 6 mit konzentrierter Salzsäure und dem Absaugen erhaltene Festsubstanz wird mit Wasser gewaschen und nochmals mit 14,0 g (62,2 mmol) Natrium-m-nitrobenzolsulfonat und 7,0 g (175 mmol) Natriumhydroxid in 280 ml Wasser unter Rühren 6 h bei Rückflußtemperatur gehalten. Nun wird wiederum bei 20 °C filtriert, das Filtrat mit konzentrierter Salzsäure auf pH 6 eingestellt und abgesaugt. Der Filterrückstand wird mit Wasser gewaschen und aus Dimethylformamid/Wasser umkristallisiert. Ausbeute: 19,0 g (38 % d. Th.) 6-(p-Aminophenyl)-5-methyl-3(2H)-pyridazinon, gelbbraune Kristalle, Schmelzpunkt 283 bis 284 °C.

Analyse für $C_{11}H_{11}N_3O$ (201,2):
Ber. C 65,7 H 5,5 N 20,9 %
Gef. C 65,5 H 5,5 N 20,9 %

Beispiel 1

6,0 g (32,0 mmol) 6-(p-Aminophenyl)-3(2H)-pyridazinon werden in 30 ml Ameisensäure unter Rühren 1 h am Rückfluß gehalten. Man trägt das Reaktionsgemisch in 500 ml Wasser ein, saugt ab, wäscht mit Wasser und kristallisiert aus Dimethylformamid/Wasser um. Man isoliert 4,0 g (58 % d. Th.) 6-(p-Formylaminophenyl)-3(2H)-pyridazinon, hellbeige Kristalle, Schmelzpunkt 300 °C.

Analyse für $C_{11}H_9N_3O_2$ (215,2):
Ber. C 61,4 H 4,2 N 19,5 O 14,9 %
Gef. C 60,7 H 4,2 N 19,3 O 15,1 %

Beispiel 2

6,0 (29,8 mmol) 6-(p-Aminophenyl)-5-methyl-3(2H)-pyridazinon und 3,8 g (35,7 mmol) Isobutyrylchlorid werden in 100 ml absolutem Tetrahydrofuran unter Rühren 6 h bei Rückflußtemperatur gehalten. Man engt ein, verrührt den Rückstand mit Wasser und saugt ab. Den Filterrückstand kristallisiert man zuerst aus Dimethylformamid/Wasser und dann noch aus Ethanol um. Ausbeute: 3,1 g (38 % d. Th.) 6-(p-Isobutyrylaminophenyl)-5-methyl-3(2H)-pyridazinon, hellgelbe Kristalle, Schmelzpunkt 245 bis 248 °C.

Analyse für $C_{15}H_{17}N_3O_2$ (271,3):
Ber. C 66,4 H 6,3 N 15,5 O 11,8 %
Gef. C 66,0 H 6,3 N 15,4 O 11,8 %

Beispiel 3

5,0 g (26,7 mmol) 6-(p-Aminophenyl)-3(2H)-pyridazinon und 3,2 g (28,3 mmol) Chloracetylchlorid werden in 150 ml absolutem Tetrahydrofuran unter Rühren 8 h bei Rückflußtemperatur gehalten. Man engt ein, verrührt den Rückstand mit Wasser und saugt ab. Die isolierte Festsubstanz wird zuerst aus Dimethylformamid/Wasser und dann noch aus Propanol umkristallisiert. Man erhält 3,9 g (55 % d. Th.) 6-(p-Chloracetylaminophenyl)-3(2H)--pyridazinon, hellbeige Kristalle, Schmelzpunkt 261 bis 262 °C.

Analyse für $C_{12}H_{10}ClN_3O_2$ (263,7):
Ber. C 54,7 H 3,8 Cl 13,4 N 15,9 %
Gef. C 54,8 H 3,9 Cl 13,3 N 16,2 %

Beispiel 4

5,0 g (24,8 mmol) 6-(p-Aminophenyl)-5-methyl-3(2H)-pyridazinon und 3,4 g (30,1 mmol) Chloracetylchlorid werden in 150 ml absolutem Tetrahydrofuran unter Rühren 8 h bei Rückflußtemperatur gehalten. Man engt ein, verrührt den Rückstand mit Wasser und saugt ab. Den Filterrückstand kristallisiert man zuerst aus Ethanol und dann noch aus Dimethylformamid/Wasser um. Man isoliert 2,9 g (42 % d. Th.) 6-(p-Chloracetylaminophenyl)-5-methyl-3(2H)--pyridazinon, beige Kristalle, Schmelzpunkt 232 bis 234 °C.

Analyse für $C_{13}H_{12}ClN_3O_2$(277,7):
Ber. C 56,2 H 4,4 Cl 12,8 N 15,1 %
Gef. C 56,5 H 4,5 Cl 12,8 N 15,5 %

Beispiel 5

Zu 5,0 g (26,7 mmol) 6-(p-Aminophenyl)-3(2H)-pyridazinon in 50 ml Aceton gibt man unter Rühren tropfenweise 3,8 g (29,9 mmol) 3-Chlorpropionylchlorid. Anschließend wird das Reaktionsgemisch noch 3 h bei Raumtemperatur nachgerührt. Man saugt bei 10 °C ab, wäscht zuerst mit Aceton, dann mit Wasser und kristallisiert aus Dimethylsulfoxid/Wasser unter Zusatz von Aktivkohle um. Die erhaltenen Kristalle werden in 100 ml Aceton unter Rühren 2 h bei Rückflußtemperatur gehalten. Es wird dann bei Raumtemperatur abgesaugt. Der Filterrückstand wird nochmals in 100 ml Aceton unter Rühren 2 h bei Rückflußtemperatur gehalten. Nach dem Absaugen bei 20 °C

und dem Trocknen im Vakuum bei 80 °C isoliert man 3,2 g (43 % d. Th.) 6-[p-(3-Chlorpropionylamino)-phenyl]-3(2H)-pyridazinon, hellgelbe Kristalle, Schmelzpunkt 300 °C.

Analyse für $C_{13}H_{12}ClN_3O_2$ (277,7):
Ber. C 56,2 H 4,4 N 15,1 O 11,5 %
Gef. C 56,4 H 4,4 N 15,1 O 11,7 %

Beispiel 6

Führt man das Beispiel 4 unter Verwendung von 2-Chlorpropionylchlorid (3,8 g (29,9 mmol)) an Stelle von Chloracetylchlorid durch, so erhält man nach dem Umkristallisieren aus Methanol/Wasser 2,8 g (39 % d. Th.) 6-[p-(2-Chlorpropionylamino)phenyl]-5-methyl-3(2H)-pyridazinon, hellgelbe Kristalle, Schmelzpunkt 226 bis 227 °C.

Analyse für $C_{14}H_{14}ClN_3O_2$ (291,7):
Ber. C 57,6 H 4,8 Cl 12,1 N 14,4 %
Gef. C 57,8 H 4,8 Cl 11,7 N 14,4 %

Beispiel 7

4,0 g (19,9 mmol) 6-(p-Aminophenyl)-5-methyl-3(2H)-pyridazinon und 3,6 g (23,9 mmol) 2-Acetoxypropionylchlorid werden in 60 ml Pyridin unter Rühren 3,5 h bei 0 °C gehalten. Man engt ein, löst den Rückstand in Methylenchlorid und wäscht die erhaltene Lösung zuerst dreimal mit je 50 ml 10prozentiger wäßriger Citronensäure und dann noch dreimal mit je 50 ml Wasser. Die Methylenchlorid-Phase wird eingeengt und der Rückstand aus Ether/Chloroform umkristallisiert. Man erhält 4,2 g (67 % d. Th.) 6-[p-(2-Acetoxypropionylamino)phenyl]-5-methyl-3(2H)-pyridazinon, hellbeige Kristalle, Schmelzpunkt 191 bis 192 °C.

Analyse für $C_{16}H_{17}N_3O_4$ (315,3):
Ber. C 60,9 H 5,4 N 13,3 O 20,3 %
Gef. C 60,5 H 5,4 N 13,3 O 20,3 %

Beispiel 8

2,0 g (6,3 mmol) 6-[p-(2-Acetoxypropionylamino)phenyl]-5-methyl-3(2H)-pyridazinon (siehe Beispiel 7) und 20 ml 1 n Natronlauge werden in 25 ml Methanol unter Rühren 20 min bei Raumtemperatur gehalten. Die Lösung wird mit 2 n Salzsäure auf pH 6 eingestellt und anschließend mit 33 ml Wasser verdünnt. Nach dem Stehen über Nacht bei 0 °C wird abgesaugt. Der Filterrückstand wird mit Wasser gewaschen und aus Dimethylformamid/Wasser umkristallisiert. Ausbeute: 1,4 g (79 % d. Th.) 6-[p-(2-Hydroxypropionylamino)phenyl]-5-methyl-3(2H)-pyridazinon-viertelhydrat, hellbraune Kristalle, Schmelzpunkt 194 bis 195 °C.

Analyse für $C_{14}H_{15}N_3O_3 \cdot 0,25\ H_2O$ (277,8):
Ber. C 60,5 H 5,6 N 15,1 O 18,7 %
Gef. C 60,5 H 5,5 N 15,0 O 18,5 %

Beispiel 9

Zu 6,0 g (32,0 mmol) 6-(p-Aminophenyl)-3(2H)-pyridazinon und 3,3 g (32,6 mmol) Triethylamin in 100 ml Aceton werden unter Rühren tropfenweise 4,5 g (36,7 mmol) 2-Methoxypropionylchlorid gegeben. Nach beendeter Zugabe wird das Reaktionsgemisch noch 16 h bei Raumtemperatur gerührt. Man saugt bei 10 °C ab, wäscht zuerst mit Aceton, dann mit Wasser und kristallisiert aus Dimethylformamid/Toluol um. Ausbeute: 2,0 g (23 % d. Th.) 6-[p-(2-Methoxypropionylamino)phenyl]-3-(2H)-pyridazinon, leicht gelbliche Kristalle, Schmelzpunkt 208 bis 210 °C.

Analyse für $C_{14}H_{15}N_3O_3$ (273,3):
Ber. C 61,5 H 5,5 N 15,4 O 17,6 %
Gef. C 61,3 H 5,4 N 15,3 O 17,5 %

Beispiel 10

Zu 4,6 g (24,6 mmol) 6-(p-Aminophenyl)-3(2H)-pyridazinon in 100 ml absolutem Tetrahydrofuran werden unter Rühren tropfenweise 5,1 g (49,3 mmol) Cyanacetylchlorid gegeben. Nach beendeter Zugabe wird das Reaktionsgemisch noch 20 h bei Raumtemperatur gerührt. Man saugt bei 10 °C ab, wäscht mit Wasser und kristallisiert aus Dimethylformamid/Wasser um. Man isoliert 3,7 g (59 % d. Th.) 6-(p-Cyanacetylaminophenyl)-3(2H)-pyridazinon, beige Kristalle, Schmelzpunkt 290 bis 292 °C.

Analyse für $C_{13}H_{10}N_4O_2$ (254,2):
Ber. C 61,4 H 4,0 N 22,0 O 12,6 %
Gef. C 61,1 H 3,7 N 22,0 O 12,6 %

Beispiel 11

4,0 g (21,4 mmol) 6-(p-Aminophenyl)-3(2H)-pyridazinon und 2,7 g (25,8 mmol) Cyclopropancarbonsäurechlorid werden in 150 ml absolutem Tetrahydrofuran unter Rühren 15 h bei Rückflußtemperatur gehalten. Man

engt ein, verrührt den Rückstand mit Wasser und saugt ab. Nach dem Umkristallisieren aus Dimethylformamid/Wasser erhält man 3,9 g (71 % d. Th.) 6-(p-Cyclopropylcarbonylaminophenyl)-3(2H)-pyridazinon, beige Kristalle, Schmelzpunkt 298 bis 299 °C.

Analyse für $C_{14}H_{13}N_3O_2$ (255,3):
Ber. C 65,9 H 5,1 N 16,5 %
Gef. C 65,5 H 5,2 N 16,6 %

Beispiel 12

6,0 g (32,0 mmol) 6-(p-Aminophenyl)-3(2H)-pyridazinon werden in 50 ml Methacrylsäureanhydrid unter Rühren 2 h bei Rückflußtemperatur gehalten. Man gießt das Reaktionsgemisch auf Eis, saugt ab, wäscht mit Wasser und kristallisiert zuerst aus Dimethylformamid/Wasser und dann noch aus Ethanol um. Ausbeute: 5,2 g (63 % d. Th.) 6-(p-Methacryloylaminophenyl)-3(2H)-pyridazinon, hellbeige Kristalle, Schmelzpunkt 253 bis 255 °C (Zers.).

Analyse für $C_{14}H_{13}N_3O_2$ (255,3):
Ber. C 65,9 H 5,1 N 16,5 %
Gef. C 66,0 H 5,0 N 16,4 %

Beispiel 13

Wird im Beispiel 12 anstatt 6-(p-Aminophenyl)-3(2H)-pyridazinon 6-(p-Aminophenyl)-5-methyl-3(2H)-pyridazinon (6,0 g (29,8 mmol)) eingesetzt, so erhält man nach dem Umkristallisieren aus Dimethylformamid/Wasser 4,7 g (58 % d. Th.) 6-(p-Methacryloylaminophenyl)-5-methyl-3(2H)-pyridazinon hellbeige Kristalle, Schmelzpunkt 245 °C.

Analyse für $C_{15}H_{15}N_3O_2$ (269,3):
Ber. C 66,9 H 5,6 N 15,6 %
Gef. C 66,3 H 5,7 N 15,3 %

Beispiel 14

Führt man das Beispiel 3 unter Verwendung von Chlorameisensäuremethylester (3,6 g (38,1 mmol)) an Stelle von Chloracetylchlorid durch, so isoliert man nach dem Umkristallisieren aus Dimethylformamid/Wasser 3,4 g (52 % d. Th.) 6-(p-Methoxycarbonylaminophenyl)-3(2H)-pyridazinon, hellgelbe Kristalle, Schmelzpunkt 260 bis 261 °C (Zers.).

Analyse für $C_{12}H_{11}N_3O_3$ (245,2):
Ber. C 58,8 H 4,5 N 17,1 %
Gef. C 58,5 H 4,7 N 17,0 %

Beispiel 15

Wird im Beispiel 4 anstatt Chloracetylchlorid Chlorameisensäuremethylester (2,8 g (29,6 mmol)) eingesetzt, so isoliert man nach dem Umkristallisieren - zuerst aus Dimethylformamid/Wasser und dann aus Ethanol - 2,0 g (31 % d. Th.) 6-(p-Methoxycarbonylaminophenyl)-5-methyl-3(2H)-pyridazinon, beige Kristalle, Schmelzpunkt 259 bis 260 °C (Zers.).

Analyse für $C_{13}H_{13}N_3O_3$ (259,3):
Ber. C 60,2 H 5,1 N 16,2 %
Gef. C 60,0 H 5,1 N 16,4 %

Beispiel 16

Führt man das Beispiel 3 unter Verwendung von Chlorameisensäure-2-chlorethylester (5,5 g (38,5 mmol)) an Stelle von Chloracetylchlorid durch, so erhält man nach dem Umkristallisieren - zuerst aus Dimethylformamid/Wasser und dann aus Ethanol - 4,7 g (60 % d. Th.) 6-[p-(2-Chlorethoxycarbonylamino)phenyl]-3(2H)-pyridazinon, beige Kristalle, Schmelzpunkt 242 bis 243 °C (Zers.).

Analyse für $C_{13}H_{12}ClN_3O_3$ (293,7):
Ber. C 53,2 H 4,1 Cl 12,1 N 14,3 %
Gef. C 53,3 H 4,2 Cl 12,0 N 14,5 %

Beispiel 17

Zu einer Lösung von 9,9 g (100 mmol) Phosgen in 100 ml absolutem Ether gibt man tropfenweise unter Rühren bei -10 °C 2,8 g (49,9 mmol) Prop-2--inol. Anschließend wird die Reaktionslösung 1 Stunde bei -10 °C gerührt. Man läßt eine weitere Stunde bei Raumtemperatur rühren und entfernt dann zuerst den Überschuß des Phosgens durch einen trockenen Strom von Stickstoff und danach unter vermindertem Druck bei Raumtemperatur den Ether. Der zurückbleibende Chlorameisensäureprop-2-inylester (5,2 g) wird unter Rühren zu 4,1 g (21,9 mmol) 6-(p-Aminophenyl)-3(2H)-pyridazinon in 150 ml absolutem Tetrahydrofuran getropft. Man läßt noch 20 h bei Raumtemperatur rühren, engt ein, verrührt den Rückstand mit Wasser und saugt ab. Die erhaltene Festsubstanz wird getrocknet und danach in 100 ml Aceton unter Rühren 2 h bei Rückflußtemperatur gehalten. Anschließend wird bei Raumtemperatur abgesaugt. Der Filterrückstand wird nochmals in 100 ml Aceton unter Rühren 2 h bei Rückflußtemperatur gehalten. Nach dem Absaugen bei 20 °C und dem Umkristallisieren aus Dimethylformamid/Wasser unter Zusatz von Aktivkohle isoliert man 0,7 g (12 % d. Th.) 6-[p-(Prop-2-inyloxycarbonylamino)phenyl]-3(2H)-pyridazinon, hellbraune Kristalle, Schmelzpunkt 268 °C (Zers.).

Analyse für $C_{14}H_{11}N_3O_3$ (269,3):
Ber. C 62,4 H 4,1 N 15,6 O 17,8 %
Gef. C 62,0 H 4,1 N 15,1 O 18,0 %

Beispiel 18

Zu 4,1 g (21,9 mmol) 6-(p-Aminophenyl)-3(2H)-pyridazinon in 150 ml absolutem Tetrahydrofuran werden unter Rühren tropfenweise 8,5 g (49,8 mmol) Chlorameisensäurebenzylester gegeben. Nach beendeter Zugabe läßt man noc 20 h bei Raumtemperatur rühren, engt ein, verrührt den Rückstand mit Wasser und saugt ab. Die erhaltene Festsubstanz wird getrocknet und danach in 100 ml Aceton unter Rühren 2 h bei Rückflußtemperatur gehalten Anschließend wird bei Raumtemperatur abgesaugt. Der Filterrückstand wird nochmals in 100 ml Aceton unter Rühren 2 h bei Rückflußtemperatur gehalten. Nach dem Absaugen bei 20 °C und dem Umkristallisieren aus Dimethylformamid/Wasser isoliert man 1,4 g (20 % d. Th.) 6-(p-Benzyloxycarbonylaminophenyl)-3(2H)-pyridazinon, beige Kristalle, Schmelzpunkt 239 bis 240 °C.

Analyse für $C_{18}H_{15}N_3O_3$ (321,3):
Ber. C 67,3 H 4,7 N 13,1 O 14,9 %
Gef. C 66,7 H 4,6 N 13,0 O 15,1 %

Beispiel 19

Wird im Beispiel 18 anstatt Chlorameisensäurebenzylester Chlorameisensäurephenylester (7,8 g (49,8 mmol)) eingesetzt, so isoliert man nach dem Umkristallisieren aus Dimethylformamid/Wasser 2,3 g (34 % d. Th.) 6-(p-Phenoxycarbonylaminophenyl)-3(2H)-pyridazinon-viertelhydrat, leicht gelbliche Kristalle, Schmelzpunkt 228 bis 230 °C.

Analyse für $C_{17}H_{13}N_3O_3 \cdot 0,25\ H_2O$ (311,8):
Ber. C 65,5 H 4,4 N 13,5 O 16,7 %
Gef. C 65,9 H 4,4 N 13,0 O 16,3 %

Beispiel 20

6,0 g (32,0 mmol) 6-(p-Aminophenyl)-3(2H)-pyridazinon werden mit 5 Tropfen Triethylamin und 2,4 g (42,1 mmol) Methylisocyanat in 150 ml absolutem Tetrahydrofuran unter Rühren 8 h bei Rückflußtemperatur gehalten. Man saugt bei 10 °C ab, wäscht mit Wasser und kristallisiert aus Dimethylformamid/Wasser um. Ausbeute: 3,5 g (45 % d. Th.) 6-[p-(3-Methylureido)phenyl]-3(2H)-pyridazinon, farblose Kristalle, Schmelzpunkt 280 °C.

Analyse für $C_{12}H_{12}N_4O_2$ (244,3):
Ber. C 59,0 H 4,9 N 22,9 %
Gef. C 58,6 H 5,0 N 22,7 %

Beispiel 21

Wird im Beispiel 20 anstatt 6-(p-Aminophenyl)-3(2H)-pyridazinon 6-(p-Aminophenyl)-5-methyl-3(2H)-pyridazinon (6,0 g (29,8 mmol)) eingesetzt, so isoliert man nach dem Umkristallisieren aus Dimethylformamid/Wasser 2,7 g (35 % d. Th.) 6-[p-(3-Methylureido)phenyl]-5-methyl-3(2H)-pyridazinon, beige Kristalle, Schmelzpunkt 280 bis 281 °C.

Analyse für $C_{13}H_{14}N_4O_2$ (258,3):
Ber. C 60,4 H 5,5 N 21,7 %
Gef. C 60,1 H 5,4 N 21,5 %

Formulierungsbeispiele, die in üblicher Weise hergestellt werden:

I. Herstellung von Tabletten:

| | |
|---|---|
| Wirkstoff | 10 mg |
| Polyvinylpyrrolidon (mittl. M.G. 25 000) | 170 mg |
| Polyethylenglykol (mittl. M.G. 4 000) | 14 mg |
| Hydroxypropylmethylcellulose | 40 mg |
| Talkum | 4 mg |
| Magnesiumstearat | 2 mg |
| | 240 mg |

Der Wirkstoff wird mit Polyvinylpyrrolidon in 10prozentiger wäßriger Lösung befeuchtet, durch ein Sieb mit der lichten Maschenweite 1,0 mm getrieben und bei 50 °C getrocknet. Dieses Granulat wird mit Polyethylenglykol (mittl. M.G. 4 000), Hydroxypropylmethylcellulose, Talkum und Magnesiumstearat vermischt und zu Tabletten á 240 mg verpreßt.

II. Herstellung von Dragees:

| | |
|---|---|
| Wirkstoff | 10 mg |
| Lactose | 90 mg |
| Maisstärke | 60 mg |
| Polyvinylpyrrolidon | 6 mg |
| Magnesiumstearat | 1 mg |
| | 167 mg |

Die Mischung der Wirkstoffsubstanz mit Lactose und Maisstärke wird mit einer 8prozentigen wäßrigen Lösung des Polyvinylpyrrolidons durch Sieb 1,5 mm granuliert, bei 50 °C getrocknet und nochmals durch Sieb 1,0 mm getrieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

Zur Untersuchung der pharmakodynamischen Eigenschaften der erfindungsgemäßen Produkte wurden folgende Methoden verwendet:

1. Hemmung der durch Collagen induzierten Aggregation von Thrombozyten des Menschen in vitro.

Thrombozytenreiches Plasma wird durch Zentrifugation (300 g, 10 min Dauer bei 4°C) von venösem Citratblut gewonnen. Die photometrische Messung der Thrombozytenaggregation erfolgt unter Zusatz von $MgCl_2$ (Endkonzentration 10 mmol/l) und von Collagen Stago (Endkonzentratio 0,02 mg/ml) im Born-Aggregometer Mk 3. Als Aggregationsmaß findet di maximale Extinktionsänderung/sec Verwendung.

Die Prüfung der aggregationshemmenden Wirksamkeit der Substanzen wir nach einer Inkubationszeit von 10 min vorgenommen.

Als EC 50% wird die Konzentration bestimmt, welche eine 50%ige Hemmung der Aggregation verursacht.

2. Blutdrucksenkende Wirkung an der narkotisierten Ratte.

Zur Testung der blutdrucksenkenden Wirkung erhalten Gruppen von 3 bi 5 männlichen Sprague-Dawley-Ratten (240 bis 280 g) in Urethan-Narkos (1,78 mg/kg i.p.) die Substanzen intraperitoneal appliziert.

Die Messung des Blutdrucks in der Arteria carotis erfolgt über Statham-Transducer. Als ED 20% wird die Dosis bestimmt, welche den mittleren Carotisdruck um 20% senkt.

3. Magensekretionshemmung.

Die Hemmung der Magensäuresekretion kommt in einem Anstieg des pH-Wertes der Magenschleimhaut zum Ausdruck. Sie wird an Gruppen von 4 weiblichen Sprague-Dawley-Ratten (160 bis 180 g) untersucht. Die Tiere erhalten 48 Stunden kein Futter (Wasser ad libitum) und werden mit unterschiedlichen Dosen der Prüfsubstanzen (p.o.) vorbehandelt. Nach 1 Stunde werden sie mit Hexobarbital-Na (4,64 mg/kg i.v.) narkotisiert. Anschließend wird eine pH-Elektrode (Philips-Spezialelektrode Typ CJP) in den Magen eingeführt und der pH-Wert auf der Oberfläche der Magenschleimhaut gemessen (pH-Wert bei unbehandelten Tieren: 1,40 ÷ 0,002; N = 200). Aus der linearen Regression zwischen den Logarithmen der applizierten Dosen und der Zunahme des pH-Wertes wird als ED 0,75 die Dosis bestimmt, die im Vergleich zu unbehandelten Kontrolltieren eine pH-Zunahme um durchschnittlich 0,75 bewirkt.

Die erfindungsgemäßen Substanzen - gekennzeichnet durch aggregationshemmende Wirkung auf Thrombozyten sowie blutdrucksenkende Wirkung - zeigen ihre pharmakologischen Effekte in Dosen unter 1,0 mg/l bzw. 1 mg/kg und sind damit deutlich wirksamer als die bekannten Substanzen. Außerdem vermindern die Verbindungen die Säuresektretion des Magens.

## Patentansprüche

1. 6-Aryl-3(2H)-pyridazinone der Formel I,

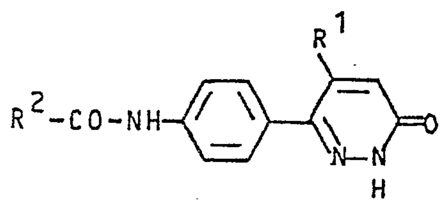

(I)

in der
$R^1$ ein Wasserstoffatom oder einen Alkylrest mit 1 bis 3 C-Atomen und
$R^2$ ein Wasserstoffatom,
einen Alkylrest mit 1 bis 8 C-Atomen, der durch 1 bis 4 Halogenatome, eine Hydroxygruppe, eine Alkoxygruppe mit 1 bis 4 C-Atomen im Alkyl, eine Alkanoyloxygruppe mit 1 bis 5 C-Atomen im Alkanoylrest oder eine Cyangruppe substituiert ist,
einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist,
einen Alkenylrest mit 2 bis 8 C-Atomen,
eine Gruppe der Formel $-X-R^3$, in der X für ein Sauerstoffatom oder ein Schwefelatom und $R^3$ für einen Alkylrest mit 1 bis 8 C-Atomen, der substituiert sein kann durch 1 bis 4 Halogenatome, eine Alkoxygruppe mit 1 bis 4 C-Atomen im Alkyl, die mindestens 2 C-Atome von X entfernt ist, oder einen Phenylrest, der seinerseits gegebenenfalls 1 bis 3 gleiche oder verschiedene Substituenten aus der Gruppe Alkyl mit 1 bis 3 C-Atomen, Alkoxy mit 1 bis 3 C-Atomen im Alkyl, Halogen, Trifluormethyl oder Nitro aufweist, einen Cycloalkylrest mit 3 bis 8 C-Atomen im Ring, der gegebenenfalls ein- bis vierfach durch Alkylreste mit 1 bis 4 C-Atomen substituiert ist, einen Alkenylrest mit 3 bis 8 C-Atomen, einen Alkinylrest mit 3 bis 8 C-Atomen oder einen Phenylrest, der gegebenenfalls durch 1 bis 3 gleiche oder verschiedene Reste aus der Gruppe Alkyl mit 1 bis 4 C-Atomen, Alkoxy mit 1 bis 4 C-Atomen im Alkyl, Halogen, Trifluormethyl, Cyan oder Nitro substituiert ist,stehen,
oder eine Gruppe der Formel $-NH-R^4$, in der $R^4$ für einen Alkylrest mit 1 bis 8 C-Atomen steht, bedeuten,
und $R^2$ zusätzlich, wenn $R^1$ für einen Alkylrest mit 1 bis 3 C-Atomen steht, einen unsubstituierten Alkylrest mit 2 bis 8 C-Atomen bedeutet.

2. 6-Aryl-3(2H)-pyridazinone der Formel I nach Anspruch 1, dadurch gekennzeichnet, daß $R^1$ ein Wasserstoffatom oder Methyl bedeutet und $R^2$ folgende Reste darstellt:

einen Halogenalkylrest mit 1 bis 4 C-Atomen und 1 Chloratom;
einen Alkoxyalkylrest mit 1 bis 3 C-Atomen im Alkylrest und 1 bis 2 C-Atomen im Alkylenrest;
einen Alkenylrest mit 2 bis 4 C-Atomen;
eine Gruppe der Formel $-O-R^3$, wobei $R^3$ folgende Reste darstellt:
einen unsubstituierten Alkylrest mit 1 bis 4 C-Atomen;
einen Halogenalkylrest mit 1 bis 4 C-Atomen und 1 Chloratom;
eine Gruppe der Formel $-NH-R^4$, in der $R^4$ für eine Alkylgruppe mit 1 bis 4 C-Atomen steht.

3. 6-Aryl-3(2H)-pyridazinone der Formel I nach Anspruch 1, dadurch gekennzeichnet, daß $R^1$ ein Wasserstoffatom oder Methyl bedeutet und $R^2$ folgende Reste darstellt: Chlormethyl, 2-Chlorethyl, 1-Methoxyethyl, 2-Propenyl, Methoxy, 2-Chlorethoxy oder die Methylaminogruppe.

4. 6-Aryl-3(2H)-pyridazinone der Formel I nach Anspruch 1, dadurch gekennzeichnet, daß $R^1$ ein Wasserstoffatom bedeutet und $R^2$ folgende Reste darstellt: Chlormethyl, 1-Methoxyethyl, Methoxy, 2-Chlorethoxy oder die Methylaminogruppe.

5. Verfahren zur Herstellung der neuen Verbindungen der Formel I nach Anspruch 1, in denen $R^2$ für ein Wasserstoffatom, einen Alkylrest mit 1 bis 8 C-Atomen, der durch 1 bis 4 Halogenatome, eine Alkoxygruppe mit 1 bis 4 C-Atomen im Alkyl, eine Alkanoyloxygruppe mit 1 bis 5 C-Atomen im Alkanoylrest oder eine Cyangruppe substituiert ist, einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist, einen Alkenylrest mit 2 bis 8 C-Atomen oder (wenn $R^1$ $C_1$- bis $C_3$-Alkyl bedeutet) einen Alkylrest mit 2 bis 8 C-Atomen steht, dadurch gekennzeichnet, daß man eine Verbindung der Formel II,

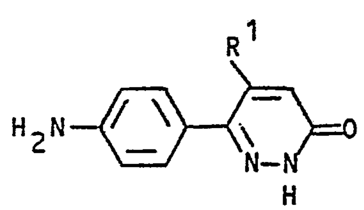

(II)

in der $R^1$ die für Formel I angegebenen Bedeutungen hat, mit einem Acylierungsmittel der Formel III,

$$R^5\text{-CO-Y} \qquad (III)$$

in der $R^5$ ein Wasserstoffatom, einen Alkylrest mit 1 bis 8 C-Atomen, der durch 1 bis 4 Halogenatome, eine Alkoxygruppe mit 1 bis 4 C-Atomen im Alkyl, eine Alkanoyloxygruppe mit 1 bis 5 C-Atomen im Alkanoylrest oder eine Cyangruppe substituiert ist, einen Cycloalkylrest mit 3 bis 8 C-Atomen im Ring, der gegebenenfalls ein- bis vierfach durch Halogenatome und/oder Alkylreste mit 1 bis 4 C-Atomen substituiert ist, einen Alkenylrest mit 2 bis 8 C-Atomen oder (wenn $R^1$ = $C_1$- bis $C_3$-Alkyl) einen Alkylrest mit 2 bis 8 C-Atomen bedeutet, und Y für OH, ein Halogenatom, insbesondere ein Chloratom, einen niederen Alkoxyrest oder $-O-CO-R^5$, wobei $R^5$ die für Formel III angegebenen Bedeutungen hat, steht, in an sich bekannter Weise umsetzt.

6. Verfahren zur Herstellung der neuen Verbindungen der Formel I nach Anspruch 1, in denen $R^2$ für einen Hydroxyalkylrest steht, dadurch gekennzeichnet, daß man eine Verbindung der Formel I, in der $R^2$ einen Alkanoyloxyalkylrest bedeutet, nach an sich bekannten Methoden, z. B mit einer Base wie Natriumhydroxid, in einem Gemisch aus Wasser und einem niederen Alkohol, wie Methanol, Ethanol oder Propanol, bei Temperaturen von 0 bis 50 °C selektiv hydrolysiert.

7. Verfahren zur Herstellung der neuen Verbindungen der Formel I nach Anspruch 1, in denen $R^2$ für eine Gruppe der Formel $-X-R^3$ steht, dadurch gekennzeichnet, daß man ein Aminophenyl-pyridazinon der Formel II,

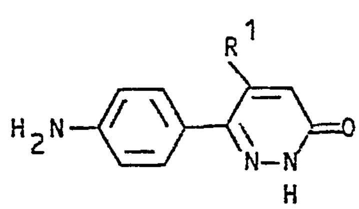

(II)

in der $R^1$ die für Formel I angegebenen Bedeutungen hat, mit einer Verbindung der Formel IV,

$$R^3\text{-X-CO-Z} \qquad (IV)$$

in der X und $R^3$ die gleichen Bedeutungen haben wie in der Gruppe der Formel $-X-R^3$, die für den Rest $R^2$ der Verbindungen der Formel I

stehen kann, und Z für ein Halogenatom steht, in an sich bekannter Weise umsetzt.

8. Verfahren zur Herstellung der neuen Pyridazinone der Formel I nach Anspruch 1, in denen $R^2$ für eine Gruppe der Formel $-NH-R^4$ steht, dadurch gekennzeichnet, daß man eine Verbindung der Formel II,

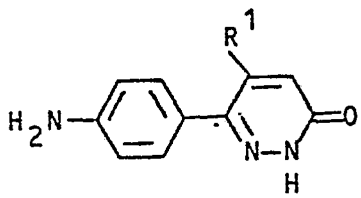

(II)

in der $R^1$ die für Formel I angegebenen Bedeutungen hat, mit einem Isocyanat der Formel V,

$$R^4-N=C=O \qquad (V)$$

in der $R^4$ die gleiche Bedeutung hat wie in der Gruppe der Formel $-NH-R^4$, die für den Rest $R^2$ der Verbindungen der Formel I stehen kann, in an sich bekannter Weise umsetzt.

9. Verfahren zur Herstellung derjenigen neuen Verbindungen der Formel I nach Anspruch 1, in denen $R^2$ für einen Alkylrest mit 1 bis 8 C-Atomen steht, der durch eine Alkoxygruppe mit 1 bis 4 C-Atomen im Alkyl oder eine Cyangruppe substituiert ist, dadurch gekennzeichnet, daß man in einer Verbindung der Formel I, in der $R^2$ für einen Alkylrest mit 1 bis 8 C-Atomen steht, der durch ein Chlor-, ein Brom- oder ein Iodatom substituiert ist, das Halogenatom in üblicher Weise gegen eine Alkoxy- oder eine Cyangruppe austauscht.

10. Verfahren zur Herstellung der neuen Verbindungen der Formel I nach Anspruch 1, in denen $R^1$ für ein Wasserstoffatom und $R^2$ für ein Wasserstoffatom oder einen Cycloalkylrest mit 3 bis 8 C-Atomen im Ring, der gegebenenfalls ein- bis vierfach durch Alkylreste mit 1 bis 4 C-Atomen substituiert ist, stehen, dadurch gekennzeichnet, daß man ein Anilid der Formel VI,

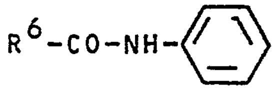

(VI)

in der $R^6$ ein Wasserstoffatom oder einen Cycloalkylrest mit 3 bis 8 C-Atomen im Ring, der gegebenenfalls ein- bis vierfach durch Alkylreste mit 1 bis 4 C-Atomen substituiert ist, bedeutet, mit Maleinsäureanhydrid in Gegenwart einer Lewis-Säure, unter den Bedingungen einer Friedel-Crafts- Acylierung umsetzt und die dabei erhaltene Acrylsäure der Formel VII,

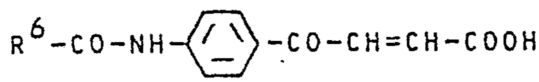 (VII)

in der $R^6$ die für Formel VI angegebenen Bedeutungen hat, mit Hydrazin cyclisiert.

11. Verfahren zur Herstellung der neuen Verbindungen der Formel I nach Anspruch 1, in denen $R^1$ eine Methylgruppe und $R^2$ ein Wasserstoffatom, einen Alkylrest mit 2 bis 8 C-Atomen oder einen Cycloalkylrest mit 3 bis 8 C-Atomen im Ring, der gegebenenfalls ein- bis vierfach durch Alkylreste mit 1 bis 4 C-Atomen substituiert ist, bedeuten, dadurch gekennzeichnet, daß man ein Anilid der Formel VIII,

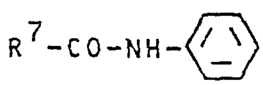 (VIII)

in der $R^7$ für ein Wasserstoffatom, einen Alkylrest mit 2 bis 8 C-Atomen oder einen Cycloalkylrest mit 3 bis 8 C-Atomen im Ring, der gegebenenfalls ein- bis vierfach durch Alkylreste mit 1 bis 4 C-Atomen substituiert ist, steht, mit Citraconsäureanhydrid in Gegenwart einer Lewis-Säure unter den Bedingungen einer Friedel-Crafts-Acylierung umsetzt und die dabei erhaltene Crotonsäure der Formel IX,

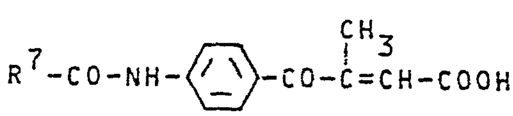 (IX)

in der $R^7$ die für Formel VIII angegebenen Bedeutungen hat, mit Hydrazin cyclisiert.

12. Arzneimittel zur Behandlung thrombotischer Erkrankungen und hohen Blutdrucks sowie zur Behandlung von Krankheitszuständen, die mit einer pathologisch gesteigerten Magensekretion einhergehen, enthaltend neben üblichen galenischen Hilfsstoffen eine wirksame Menge einer Verbindung der Formel I nach Anspruch 1 als Wirkstoff.

13. Arzneimittel zur Behandlung thrombotischer Erkrankungen und hohen Blutdrucks sowie zur Behandlung von Krankheitszuständen, die mit einer pathologisch gesteigerten Magensekretion einhergehen, enthaltend neben üblichen galenischen Hilfsstoffen eine wirksame Menge einer Verbindung der Formel I nach Anspruch 2 als Wirkstoff.

14. Verwendung eines Arzneimittels nach Anspruch 12 zur Behandlung thrombotischer Erkrankungen und hohen Blutdrucks sowie zur Behandlung von Krankheitszuständen, die mit einer pathologisch gesteigerten Magensekretion einhergehen.

0116853

Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 84100422.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE - A1 - 3 033 702 (BASF)<br>* Ansprüche 1,4; Seite 27, Zeilen 31-35 *<br>-- | 1,5,7, 12,13 | C 07 D 237/14<br>A 61 K 31/50 |
| D,A | DE - A1 - 3 022 177 (BASF)<br>* Ansprüche 1,4; Seite 27, Zeilen 26-31 *<br>-- | 1,5,7, 10,12, 13 | |
| D,A | DE - A1 - 3 022 176 (BASF)<br>* Ansprüche 1,4; Seite 14, Zeilen 1-5 *<br>-- | 1,5,12, 13 | |
| D,A | DE - A1 - 2 854 191 (BASF)<br>* Anspruch 1; Seite 5, Zeilen 1-35; Seite 8, Zeilen 12-16 *<br>-- | 1,5,12, 13 | |
| | | | RECHERCHIERTE SACHGEBIETE (Int. Cl. 3)<br>C 07 D 237/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-13
Unvollständig recherchierte Patentansprüche: -
Nicht recherchierte Patentansprüche: 14
Grund für die Beschränkung der Recherche:

Anspruch 14: Verfahren zur therapeutischen Behandlung des menschlichen oder tierischen Körpers Artikel 52(4)

| Recherchenort | Abschlußdatum der Recherche | Prüfer |
|---|---|---|
| WIEN | 27-04-1984 | LUX |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

0116853

Europäisches Patentamt

# EUROPÄISCHER TEILRECHERCHENBERICHT

Nummer der Anmeldung

EP 84100422.9

| EINSCHLÄGIGE DOKUMENTE | | | KLASSIFIKATION DER ANMELDUNG (Int Cl.3) |
|---|---|---|---|
| Kategorie | Kennzeichnung des Dokuments mit Angabe, soweit erforderlich, der maßgeblichen Teile | betrifft Anspruch | |
| A | US - A - 4 053 601 (COATES et al.)<br>* Anspruch 1; Spalte 11, Zeile 55 - Spalte 12, Zeile 48 *<br>---- | 1,12, 13 | |
| | | | RECHERCHIERTE SACHGEBIETE (Int. Cl.3) |